# EUROPÄISCHE PATENTANMELDUNG

(11) **EP 4 740 807 A1**
(43) Veröffentlichungstag der Anmeldung: **13.05.2026**
(21) Anmeldenummer: 24212326.3
(22) Anmeldetag: 12.11.2024
(51) Int. Cl.: A47K 11/00, A61L 9/12, A61L 9/14, E03D 9/00, E04H 1/12, A45D 34/02

(54) **SANITÄRKABINE UND VERFAHREN ZUM BETRIEB EINER SANITÄRKABINE**

(71) Anmelder: TOI TOI & DIXI Group GmbH, 40880 Ratingen (DE)
(72) Erfinder: WIRTZ, Holger Martin, 41468 Neuss (DE); WORIESCHECK, Tim, 26160 Bad Zwischenahn (DE); BONEWITZ, André, 36289 Friedewald (DE); ROST, Jonas, 99837 Werra-Suhl-Tal (DE)
(74) Vertreter: Andrejewski - Honke Patent- und Rechtsanwälte Partnerschaft mbB

(57) **Zusammenfassung**

Die Erfindung betrifft eine Sanitärkabine mit zumindest einem Bodenteil, zumindest einem Deckenteil, einer Mehrzahl von Seitenwandteilen und mit zumindest einer verschwenkbaren Kabinentür. Die Sanitärkabine weist fernerhin zumindest eine Beduftungseinrichtung mit zumindest einem Hauptkörper, zumindest einem Auslöseelement und zumindest einem Duftmittelbehältnis auf. Die Beduftungseinrichtung ist mit der Maßgabe im Innenraum der Sanitärkabine angeordnet, dass das Auslöseelement beim Schließen und/oder Öffnen der Kabinentür das Duftmittelbehältnis derart beaufschlagt, dass von der Beduftungseinrichtung ein Duftmittel in die Sanitärkabine abgebbar ist bzw. abgegeben wird.

## Beschreibung

Die Erfindung betrifft eine Sanitärkabine, insbesondere eine Toilettenkabine, mit zumindest einem Bodenteil, zumindest einem Deckenteil, einer Mehrzahl von Seitenwandteilen und mit zumindest einer verschwenkbaren Kabinentür. Darüber hinaus betrifft die Erfindung ein Verfahren zum Betrieb einer solchen Sanitärkabine.

Empfohlenermaßen handelt es sich bei der erfindungsgemäßen Sanitärkabine um eine mobile bzw. transportable Sanitärkabine. Ganz besonders bevorzugt handelt es sich bei der erfindungsgemäßen Sanitärkabine um eine mobile Toilettenkabine. Mobile Sanitärkabinen bzw. mobile Toilettenkabinen können insbesondere auf Transportfahrzeugen transportiert werden. Sie sind flexibel an Orten einsetzbar, an denen keine fest installierten Sanitäranlagen vorhanden sind. Die mobilen Sanitärkabinen werden daher in der Regel für bestimmte Zeiträume beispielsweise auf Baustellen oder bei Veranstaltungen wie Märkten, Messen, Festivals, Feiern und dergleichen aufgestellt und anschließend wieder abtransportiert und endgereinigt. Wenn die Sanitärkabinen für längere Zeit an einem Ort aufgestellt bleiben, finden üblicherweise Zwischenreinigungen statt, bei denen in der Regel die Fäkalientanks der Sanitärkabinen entleert und die Kabinen gereinigt werden.

Sanitärkabinen der vorstehend beschriebenen Art sind aus der Praxis in unterschiedlichen Ausführungsformen grundsätzlich bekannt und haben sich auch bewährt. Es hat sich aber gezeigt, dass es im Innenraum dieser Sanitärkabinen insbesondere aufgrund der üblicherweise in einem Fäkalientank aufgefangenen Fäkalien zur Entwicklung von Gerüchen kommen kann. Diese Gerüche können den Benutzer der Sanitärkabine stören. Es ist in diesem Zusammenhang bereits bekannt, in den Fäkalientanks der Sanitärkabinen Duftmittel einzusetzen. Nichtsdestoweniger besteht im Hinblick auf den Geruch im Innenraum der Sanitärkabinen weiter Verbesserungsbedarf. Beispielsweise bei warmen Außentemperaturen und/oder kurz vor der Zwischenreinigung oder Endreinigung der Sanitärkabinen hat sich gezeigt, dass Gerüche in den Sanitärkabinen auftreten können, die von einigen Benutzern als störend empfunden werden. Es ist daher wünschenswert, funktionssicher einen angenehmen Geruch im Innenraum der Sanitärkabinen zu realisieren. - Hier setzt die Erfindung ein.

Der Erfindung liegt demgegenüber das technische Problem zugrunde, eine Sanitärkabine der eingangs genannten Art anzugeben, bei der ein angenehmer Geruch im Innenraum funktionssicher realisiert werden kann und bei der insbesondere störenden Gerüchen im Innenraum der Sanitärkabine effektiv und funktionssicher entgegengewirkt wird und zwar insbesondere auch bei warmen Außentemperaturen und/oder kurz vor der Zwischenreinigung oder Endreinigung. Darüber hinaus liegt der Erfindung das technische Problem zugrunde, ein Verfahren zum Betrieb einer solchen Sanitärkabine anzugeben.

Zur Lösung des technischen Problems lehrt die Erfindung eine Sanitärkabine, insbesondere eine Toilettenkabine, mit zumindest einem Bodenteil, zumindest einem Deckenteil, einer Mehrzahl von Seitenwandteilen und mit zumindest einer verschwenkbaren Kabinentür, wobei die Sanitärkabine fernerhin zumindest eine Beduftungseinrichtung mit zumindest einem Hauptkörper, zumindest einem - vorzugsweise als Auslösebügel ausgestalteten - Auslöseelement und zumindest einem Duftmittelbehältnis aufweist, wobei die Beduftungseinrichtung mit der Maßgabe im Innenraum der Sanitärkabine angeordnet ist, dass das Auslöseelement beim Schließen und/oder Öffnen der Kabinentür das Duftmittelbehältnis, vorzugsweise eine Abgabeeinrichtung des Duftmittelbehältnisses, derart beaufschlagt, dass ein Duftmittel von der Beduftungseinrichtung in die Sanitärkabine abgebbar ist bzw. abgegeben wird und wobei dazu bevorzugt das Auslöseelement durch Zusammenwirkung mit der Kabinentür betätigbar ist bzw. betätigt wird.

Gemäß bevorzugter Ausführungsform ist die Beduftungseinrichtung somit mit der Maßgabe im Innenraum der Sanitärkabine angeordnet, dass das Auslöseelement beim Schließen und/oder Öffnen der Kabinentür durch Zusammenwirkung mit der Kabinentür betätigbar ist bzw. betätigt wird und wobei das Auslöseelement dabei das Duftmittelbehältnis, vorzugsweise eine Abgabeeinrichtung des Duftmittelbehältnisses, derart beaufschlagt, dass von der Beduftungseinrichtung ein Duftmittel in die Sanitärkabine abgebbar ist bzw. abgegeben wird.

Gemäß bevorzugter Ausführungsform der Erfindung ist die Abgabeeinrichtung als Sprüheinrichtung, insbesondere als Sprühkopf, ausgebildet und wobei das Duftmittel vorzugsweise in die Sanitärkabine sprühbar ist bzw. gesprüht wird. Bevorzugt ist das Duftmittelbehältnis eine Duftmittelflasche, die sehr bevorzugt einen Sprühkopf als Abgabeeinrichtung aufweist.

Hier und nachfolgend wird insbesondere auf den vertikalen Aufstellzustand der erfindungsgemäßen Sanitärkabine Bezug genommen, in dem das Bodenteil auf einem Aufstelluntergrund angeordnet ist und in dem sich das Deckenteil oberhalb des Bodenteils befindet. Es wurde bereits eingangs darauf hingewiesen, dass die erfindungsgemäße Sanitärkabine nach bevorzugter Ausführungsform eine mobile bzw. transportable Sanitärkabine, insbesondere eine mobile bzw. transportable Toilettenkabine, ist. Somit ist die erfindungsgemäße Sanitärkabine zweckmäßigerweise relativ kompakt ausgebildet und es liegt in diesem Zusammenhang im Rahmen der Erfindung, dass das Volumen des Innenraums der Sanitärkabine lediglich 1 bis 7 m³, bevorzugt 1,25 bis 3,5 m³, besonders bevorzugt 1,25 bis 3 m³ beträgt. Es ist bevorzugt, dass die erfindungsgemäße Sanitärkabine in der Draufsicht rechteckig, insbesondere quadratisch bzw. im Wesentlichen quadratisch, ausgebildet ist. Grundsätzlich liegen bezüglich der Draufsicht auf die Sanitärkabine aber auch andere Geometrien im Rahmen der Erfindung.

Dass die Abgabeeinrichtung ein Duftmittel in die Sanitärkabine abgibt, meint im Rahmen der Erfindung insbesondere, dass die Abgabeeinrichtung das Duftmittel in die Sanitärkabine sprüht und/oder tropft. Der Ausdruck Sprühen meint im Rahmen der Erfindung insbesondere auch die Abgabe in Form eines Strahls. Bevorzugt ist die Abgabeeinrichtung als Sprüheinrichtung, sehr bevorzugt als Sprühkopf, ausgebildet und sprüht das Duftmittel in die Sanitärkabine.

Gemäß bevorzugter Ausführungsform der Erfindung weist die Sanitärkabine ein Bodenteil, ein Deckenteil, drei Seitenwandteile und eine verschwenkbare Kabinentür auf. Die verschwenkbare Kabinentür bildet vorzugsweise die vierte Seitenwand der Sanitärkabine. Es ist auch möglich, dass die verschwenkbare Kabinentür Teil eines vierten Seitenwandteils ist. Die Sanitärkabine weist gemäß weiter bevorzugter Ausführungsform eine Türöffnung auf, durch die die Sanitärkabine betretbar ist und die im geschlossenen Zustand der Kabinentür von der Kabinentür verschlossen ist.

Erfindungsgemäß beaufschlagt das Auslöseelement beim Schließen und/oder Öffnen der Kabinentür das Duftmittelbehältnis, vorzugsweise eine Abgabeeinrichtung des Duftmittelbehältnisses. Das Auslöseelement beaufschlagt gemäß einer bevorzugten Ausführungsform beim Schließen und/oder Öffnen der Kabinentür die Abgabeeinrichtung, insbesondere die Sprüheinrichtung bzw. den Sprühkopf. Es ist auch möglich, dass das Auslöseelement beim Schließen und/oder Öffnen der Kabinentür eine - insbesondere der Abgabeeinrichtung gegenüberliegend angeordnete - Unterseite des Duftmittelbehältnisses beaufschlagt. Zweckmäßigerweise wird dadurch indirekt die Abgabeeinrichtung beaufschlagt, beispielsweise indem die Abgabeeinrichtung dabei gegen eine Anschlagfläche der Beduftungseinrichtung, insbesondere des Hauptkörpers der Beduftungseinrichtung gedrückt wird. Gemäß bevorzugter Ausführungsform ist das Auslöseelement - insbesondere der Auslösebügel - beim Schließen und/oder Öffnen der Kabinentür durch Zusammenwirkung mit der Kabinentür betätigbar bzw. wird betätigt. Schließen der Kabinentür meint im Rahmen der Erfindung insbesondere den Schließvorgang der Kabinentür, bei dem die verschwenkbare Kabinentür von einer geöffneten Position, in der die Sanitärkabine vorzugsweise durch die Türöffnung betretbar ist, in eine geschlossene Position, in der die Türöffnung bevorzugt von der Kabinentür verschlossen ist, verschwenkt wird. Öffnen der Kabinentür meint demgegenüber insbesondere den Öffnungsvorgang der Kabinentür.

Zusammenwirkung des Auslöseelementes mit der Kabinentür meint insbesondere eine direkte und/oder indirekte Zusammenwirkung des Auslöseelementes mit der Kabinentür. Gemäß einer bevorzugten Ausführungsform wirkt die Kabinentür - insbesondere die dem Innenraum der Sanitärkabine zugewandte Innenseite der Kabinentür - durch direkten Kontakt mit dem Auslöseelement beim Schließen und/oder Öffnen zusammen und betätigt bevorzugt auf diese Weise das Auslöseelement. Grundsätzlich liegt es aber auch im Rahmen der Erfindung, dass zwischen der Kabinentür und dem Auslöseelement ein Zwischenelement angeordnet ist, sodass das Auslöseelement indirekt durch Zusammenwirkung mit der Kabinentür betätigbar ist bzw. betätigt wird. Die Beduftungseinrichtung ist zweckmäßigerweise in der Nähe der verschwenkbaren Kabinentür im Innenraum der Sanitärkabine angeordnet. Das wird unten stehend noch näher erläutert.

Das Duftmittelbehältnis ist bevorzugt eine Duftmittelflasche. Gemäß bevorzugter Ausführungsform weist die Duftmittelflasche eine Abgabeeinrichtung, insbesondere eine Sprüheinrichtung, vorzugsweise in Form eines Sprühkopfes auf. Die Abgabeeinrichtung, insbesondere die Sprüheinrichtung, des Duftmittelbehältnisses, insbesondere der Duftmittelflasche, weist besonders bevorzugt eine Düse auf, aus der das Duftmittel abgegeben wird. Zweckmäßigerweise wird im Zuge der Beaufschlagung des Duftmittelbehältnisses, vorzugsweise der Abgabeeinrichtung bzw. der Sprüheinrichtung, durch das Auslöseelement die Abgabeeinrichtung, insbesondere die Sprüheinrichtung, heruntergedrückt und es wird Duftmittel aus dem Duftmittelbehältnis gepumpt, das insbesondere in Form eines Sprühstoßes und/oder eines Strahls und/oder von Tropfen aus der Abgabeeinrichtung, insbesondere aus der Sprüheinrichtung, abgegeben wird.

Bei dem Duftmittel handelt es sich im Übrigen bevorzugt um zumindest ein Duftöl und/oder Duftölgemisch, bevorzugt um zumindest ein Duftölgemisch enthaltend zumindest ein Duftöl und Ethanol und/oder Wasser. Es ist bevorzugt, dass im Rahmen einer Beaufschlagung des Duftmittelbehältnisses, vorzugsweise der Abgabeeinrichtung des Duftmittelbehältnisses, durch das Auslöseelement von der Abgabeeinrichtung 0,05 bis 0,5 mL, bevorzugt 0,08 bis 0,35 mL, besonders bevorzugt 0,1 bis 0,25 mL Duftmittel abgegeben werden.

Es ist weiter bevorzugt, dass das Duftmittelbehältnis ein Fassungsvermögen von 30 mL bis 250 mL, bevorzugt von 50 mL bis 150 mL, sehr bevorzugt von 75 mL bis 125 mL, ganz besonders bevorzugt von 90 mL bis 110 m L aufweist.

Das Auslöseelement, das beim Schließen und/oder Öffnen der Kabinentür erfindungsgemäß das Duftmittelbehältnis, vorzugsweise eine Abgabeeinrichtung des Duftmittelbehältnisses, derart beaufschlagt, dass ein Duftmittel in die Sanitärkabine abgebbar ist bzw. abgegeben wird, ist gemäß bevorzugter Ausführungsform als Auslösebügel ausgestaltet. Sehr bevorzugt weist das Auslöseelement, vorzugsweise der Auslösebügel, einen Beaufschlagungsabschnitt, der für die Beaufschlagung des Duftmittelbehältnisses, vorzugsweise der Abgabeeinrichtungdes Duftmittelbehältnisses, vorgesehen ist, und/oder einen Zusammenwirkungsabschnitt, der für die Zusammenwirkung mit der Kabinentür vorgesehen ist, auf. Zweckmäßigerweise sind der Beaufschlagungsabschnitt und der Zusammenwirkungsabschnitt gewinkelt zueinander angeordnet und insbesondere im rechten Winkel bzw. im Wesentlichen im rechten Winkel zueinander angeordnet.

Eine bevorzugte Ausführungsform der erfindungsgemäßen Sanitärkabine ist dadurch gekennzeichnet, dass das Auslöseelement, insbesondere der Auslösebügel, an dem Hauptkörper der Beduftungseinrichtung angeordnet ist, vorzugsweise angelenkt ist, und bevorzugt beim Schließen und/oder Öffnen der Kabinentür - insbesondere durch Zusammenwirkung mit der Kabinentür - von einer Warteposition in eine Auslöseposition überführbar ist bzw. überführt wird, vorzugsweise verschwenkbar ist bzw. verschwenkt wird, wobei das Auslöseelement, insbesondere der Auslösebügel, vorzugsweise bei der Überführung bzw. Verschwenkung von der Warteposition in die Auslöseposition das Duftmittelbehältnis, vorzugsweise die Abgabeeinrichtung des Duftmittelbehältnisses, beaufschlagt. Mit der Beaufschlagung des Duftmittelbehältnisses, vorzugsweise der Abgabeeinrichtung des Duftmittelbehältnisses, durch das Auslöseelement ist im Rahmen der Erfindung im Übrigen insbesondere das Drücken bzw. Herunterdrücken des Duftmittelbehältnisses, vorzugsweise der Abgabeeinrichtung des Duftmittelbehältnisses, durch das Auslöseelement gemeint. Die Beaufschlagung des Duftmittelbehältnisses, vorzugsweise der Abgabeeinrichtung des Duftmittelbehältnisses, erfolgt zweckmäßigerweise im Zuge der Überführung bzw. Verschwenkung des Auslöseelementes, insbesondere des Auslösebügels, von der Warteposition in die Auslöseposition. Verschwenkung des Auslöseelementes meint im Übrigen insbesondere die Verschwenkung relativ zu dem Hauptkörper der Beduftungseinrichtung. Auslöseposition meint insbesondere die Position des Auslöseelementes, in der das Duftmittelbehältnis, vorzugsweise die Abgabeeinrichtung des Duftmittelbehältnisses, zur Abgabe des Duftmittels beaufschlagt ist bzw. wurde. Warteposition meint insbesondere die Position des Auslöseelementes, in der das Duftmittelbehältnis, vorzugsweise die Abgabeeinrichtung des Duftmittelbehältnisses, auf die (nächste) Beaufschlagung zur Abgabe des Duftmittels wartet.

Es ist bevorzugt, dass das Auslöseelement für die Zusammenwirkung mit der Kabinentür und/oder für die Beaufschlagung der Sprüheinrichtung an einem Gelenkpunkt, insbesondere an lediglich einem einzigen Gelenkpunkt, an dem Hauptkörper der Beduftungseinrichtung angelenkt ist und dass vorzugsweise abgesehen von der Verschwenkbarkeit des Auslöseelementes um diesen Gelenkpunkt keine weiteren Gelenkpunkte in dem Auslöseelement und/oder zwischen dem Auslöseelement und dem Hauptkörper vorhanden sind. Die Beduftungseinrichtung zeichnet sich somit im Hinblick auf die Betätigung des Auslöseelementes und der Beaufschlagung der Sprüheinrichtung durch besondere Einfachheit und Funktionssicherheit aus.

Es wurde bereits erläutert, dass das Auslöseelement, insbesondere der Auslösebügel, vorzugsweise einen Beaufschlagungsabschnitt und einen Zusammenwirkungsabschnitt aufweist, die sehr bevorzugt gewinkelt zueinander angeordnet sind. Der Gelenkpunkt zwischen dem Hauptkörper der Beduftungseinrichtung und dem Auslöseelement ist bevorzugt in einem Endabschnitt, insbesondere an einem Endpunkt, des Beaufschlagungsabschnittes oder in einem Übergangsabschnitt, insbesondere an einem Übergangspunkt, zwischen dem Zusammenwirkungsabschnitt und dem Beaufschlagungsabschnitt angeordnet. Endabschnitt meint dabei insbesondere die letzten 10 %, vorzugsweise die letzten 5 % des Beaufschlagungsabschnittes in Bezug auf die Gesamtlänge des Beaufschlagungsabschnittes. Übergangsabschnitt meint dabei insbesondere den sich um den Übergangspunkt zwischen dem Zusammenwirkungsabschnitt und dem Beaufschlagungsabschnitt erstreckenden Abschnitt, und zwar in einer Länge von 10 %, vorzugsweise von 5 % der Gesamtlänge des Auslöseelementes.

Wenn der Gelenkpunkt zwischen dem Auslöseelement und dem Hauptkörper gemäß bevorzugter Ausführungsform in einem Endabschnitt des Beaufschlagungsabschnittes angeordnet ist, dann ist der Gelenkpunkt vorzugsweise in Bezug auf die Längserstreckung des Beaufschlagungsabschnittes hinter der Abgabeeinrichtung angeordnet. Sehr bevorzugt erfolgt die Beaufschlagung des Duftmittelbehältnisses, insbesondere der Abgabeeinrichtung, zur Abgabe von Duftmittel dann beim Schließen der Kabinentür. Wenn der Gelenkpunkt zwischen dem Auslöseelement und dem Hauptkörper gemäß bevorzugter Ausführungsform in einem Übergangsabschnitt zwischen dem Zusammenwirkungsabschnitt und dem Beaufschlagungsabschnitt angeordnet ist, dann ist der Gelenkpunkt vorzugsweise in Bezug auf die Längserstreckung des Beaufschlagungsabschnittes vor der Abgabeeinrichtung angeordnet. Sehr bevorzugt erfolgt die Beaufschlagung des Duftmittelbehältnisses, insbesondere der Abgabeeinrichtung, zur Abgabe von Duftmittel dann beim Öffnen der Kabinentür. Vorzugsweise übergreift das Auslöseelement im Übrigen die Abgabeeinrichtung. Das wird unten stehend noch näher erläutert.

Die Erfindung hat erkannt, dass störenden Gerüchen im Innenraum der Sanitärkabine durch die erfindungsgemäß im Innenraum der Sanitärkabine angeordnete Beduftungseinrichtung funktionssicher entgegengewirkt werden kann. Durch die Beaufschlagung des Duftmittelbehältnisses, insbesondere der Abgabeeinrichtung des Duftmittelbehältnisses, durch das Auslöseelement, insbesondere aufgrund der Zusammenwirkung der Kabinentür mit dem Auslöseelement, beim Schließen und/oder Öffnen der Kabinentür, erfolgt die Abgabe von Duftmittel insbesondere lediglich beim Schließen und/oder Öffnen der Kabinentür und somit vorzugsweise lediglich dann, wenn die Sanitärkabine tatsächlich benutzt wird. Das ist im Hinblick auf die Funktionssicherheit der Beduftungseinrichtung und auch bezüglich der Nutzungsdauer der Beduftungseinrichtung bis zu einem Wechsel oder der Nachfüllung des Duftmittelbehältnisses sehr vorteilhaft.

Es ist bevorzugt, dass die Beduftungseinrichtung zumindest ein Speicherelement, insbesondere einen Speicherfilz, aufweist, wobei Duftmittel, das aus der Abgabeeinrichtung abgegeben wird, in dem Speicherelement speicherbar ist bzw. gespeichert wird und wobei das Speicherelement vorzugsweise der Abgabeeinrichtung gegenüberliegend angeordnet ist, sodass die Abgabeeinrichtung bevorzugt zumindest einen Teil des Duftmittels gegen das Speicherelement abgibt, insbesondere sprüht. Gemäß bevorzugter Ausführungsform der Erfindung ist das zumindest eine Speicherelement ein Speicherfilz. Es ist zusätzlich oder alternativ möglich, dass als Speicherelement ein Schwamm und/oder ein Tuch und/oder ein Vlies eingesetzt wird. Der Ausführungsform, bei der die Beduftungseinrichtung ein Speicherelement aufweist, liegt die Erkenntnis zugrunde, dass die Beduftungseinrichtung grundsätzlich zwar nur dann Duftmittel abgibt, wenn die Sanitärkabine benutzt wird, dass das abgegebene Duftmittel aber zumindest teilweise von dem Speicherelement aufgenommen wird und dann über einen längeren Zeitraum abgegeben werden kann. Gemäß sehr bevorzugter Ausführungsform der Erfindung ist das Speicherelement der Abgabeeinrichtung, insbesondere der Sprüheinrichtung, bevorzugt einer Düse der Sprüheinrichtung, gegenüberliegend angeordnet. Ganz besonders bevorzugt ist eine Speicherelementaufnahme an dem Hauptkörper der Beduftungseinrichtung angeordnet und zwar sehr bevorzugt der Abgabeeinrichtung gegenüberliegend angeordnet. Das Speicherelement, insbesondere der Speicherfilz, kann vorzugsweise in die Speicherelementaufnahme eingeschoben werden. Sehr bevorzugt ist das Speicherelement bezüglich seiner flächigen Erstreckung vertikal bzw. im Wesentlichen vertikal in der Beduftungseinrichtung, insbesondere in der Speicherelementaufnahme, angeordnet bzw. aufgenommen.

Gemäß weiter bevorzugter Ausführungsform ist das Speicherelement beabstandet von der Abgabeeinrichtung angeordnet und der Abstand zwischen der Abgabeeinrichtung und dem Speicherelement beträgt vorzugsweise 1,0 cm bis 7,0 cm, bevorzugt 1,5 cm bis 6,5 cm, besonders bevorzugt 2,0 cm bis 6,0 cm. Abstand zwischen der Abgabeeinrichtung und dem Speicherelement meint insbesondere den kleinsten Abstand zwischen der Abgabeeinrichtung und dem Speicherelement und zwar sehr bevorzugt in der Abgaberichtung des Duftmittels. Dieser Ausführungsform liegt die Erkenntnis zugrunde, dass eine bestimmte Menge des abgegebenen Duftmittels in die Sanitärkabine abgegeben, insbesondere gesprüht, wird und dass eine bestimmte Menge des abgegebenen Duftmittels auf das Speicherelement abgeben wird, insbesondere gegen das Speicherelement gesprüht wird. Durch den Abstand zwischen der Abgabeeinrichtung und dem Speicherelement lassen sich die entsprechenden Mengen gezielt beeinflussen und insbesondere einstellen. In dem bevorzugten angegebenen Abstandsbereich ist das Verhältnis aus abgegebener Menge Duftmittel und gespeicherter Menge Duftmittel besonders vorteilhaft.

Eine bevorzugte Ausführungsform der erfindungsgemäßen Sanitärkabine ist dadurch gekennzeichnet, dass der Hauptkörper der Beduftungseinrichtung einen Auffangabschnitt, insbesondere ein Auffangreservoir, aufweist, in dem von der Abgabeeinrichtung abgegebenes Duftmittel auffangbar ist bzw. aufgefangen wird und wobei der Auffangabschnitt, insbesondere das Auffangreservoir, vorzugsweise in der Duftmittelabgaberichtung zwischen der Abgabeeinrichtung und dem Speicherelement angeordnet ist. Dieser Ausführungsform liegt die Erkenntnis zugrunde, dass in dem zumindest einen Auffangabschnitt, insbesondere in dem zumindest einen Auffangreservoir, von der Abgabeeinrichtung abgegebenes Duftmittel, insbesondere herabtropfendes Duftmittel, effektiv und funktionssicher aufgefangen werden kann. Damit wird einerseits verhindert, dass von der Abgabeeinrichtung abgegebenes bzw. herabtropfendes Duftmittel in die Sanitärkabine tropft und darüber hinaus kann aus dem Auffangabschnitt, insbesondere aus dem Auffangreservoir, über einen längeren Zeitraum Duftmittel für die Beduftung der Sanitärkabine abgegeben werden. Gemäß bevorzugter Ausführungsform ist der Auffangabschnitt, insbesondere das Auffangreservoir, in Duftmittelabgaberichtung zwischen der Abgabeeinrichtung und dem Speicherelement angeordnet. Duftmittelabgaberichtung meint insbesondere die vorgesehene Richtung, in die das Duftmittel von der Abgabeeinrichtung abgegeben, insbesondere von der Sprüheinrichtung gesprüht, wird. Auf diese Weise kann das von der Abgabeeinrichtung abgegebene Duftmittel bevorzugt einerseits zumindest teilweise von dem Speicherelement aufgenommen werden und andererseits kann herabtropfendes Duftmittel von dem Auffangabschnitt aufgenommen werden.

Sehr bevorzugt ist zumindest ein oberer Teil des Hauptkörpers der Beduftungseinrichtung, der insbesondere den Auffangabschnitt und/oder die Speicherelementaufnahme aufweist, einstückig ausgebildet. Dieser obere Teil des Hauptkörpers umgibt bevorzugt zumindest einen Abschnitt der Abgabeeinrichtung und sehr bevorzugt ist in Duftmittelabgaberichtung zunächst der Auffangabschnitt und anschließend die Speicherelementaufnahme angeordnet. Es ist sehr bevorzugt, dass das Auslöseelement der Beduftungseinrichtung an dem oberen Teil des Hauptkörpers angeordnet ist, vorzugsweise angelenkt ist.

Es ist weiter bevorzugt, dass die im montierten Zustand der Beduftungseinrichtung dem Innenraum der Sanitärkabine zugewandte Seite der Beduftungseinrichtung, insbesondere des oberen Teils des Hauptkörpers der Beduftungseinrichtung, zumindest eine Abschirmwand aufweist. Bevorzugt ist die Abschirmwand in Abgaberichtung des Duftmittels hinter der Abgabeeinrichtung vorgesehen. Sehr bevorzugt ist die Abschirmwand zwischen der Abgabeeinrichtung und dem Speicherelement, und zwar ganz besonders bevorzugt im Bereich des Auffangreservoirs, angeordnet. Dadurch wird insbesondere erreicht, dass im Zuge der Duftmittelabgabe kein Duftmittel in Richtung des Benutzers der Sanitärkabine gelangt. Das Duftmittel wird vorzugsweise lediglich in Richtung des Deckenteils und/oder zumindest eines Seitenwandteils und/oder des Speicherelementes abgegeben.

Gemäß sehr bevorzugter Ausführungsform der Erfindung ist das Auslöseelement, vorzugsweise der Auslösebügel, derart an dem Hauptkörper der Beduftungseinrichtung angeordnet, insbesondere angelenkt, dass es die Abgabeeinrichtung übergreift. Das Auslöseelement weist bevorzugt einen Beaufschlagungsfortsatz auf, der im Zuge der Betätigung des Auslöseelementes das Duftmittelbehältnis, vorzugsweise die Abgabeeinrichtung des Duftmittelbehältnisses beaufschlagt und die Abgabeeinrichtung, bevorzugt zur Abgabe des Duftmittels drückt bzw. herunterdrückt.

Das Beaufschlagen der Abgabeeinrichtung, insbesondere das Drücken bzw. Herunterdrücken der Abgabeeinrichtung kann im Rahmen der Erfindung direkt oder indirekt erfolgen. Gemäß einer Ausführungsform ist das Duftmittelbehältnis derart in der Sanitärkabine angeordnet, dass die Abgabeeinrichtung in Richtung des Deckenteils und eine der Abgabeeinrichtung gegenüberliegend angeordnete Unterseite des Duftmittelbehältnisses in Richtung des Bodenteils der Sanitärkabine orientiert ist. Dann beaufschlagt das Auslöseelement bevorzugt direkt die Abgabeeinrichtung. Gemäß einer anderen Ausführungsform ist das Duftmittelbehältnis derart in der Sanitärkabine angeordnet, dass die Abgabeeinrichtung in Richtung des Bodenteils und eine der Abgabeeinrichtung gegenüberliegend angeordnete Unterseite des Duftmittelbehältnisses in Richtung des Deckenteils der Sanitärkabine orientiert ist. Dann beaufschlagt das Auslöseelement bevorzugt die Unterseite des Duftmittelbehältnisses und sehr bevorzugt auf diese Weise indirekt Abgabeeinrichtung, indem die Abgabeeinrichtung bei der Beaufschlagung der Unterseite des Duftmittelbehältnisses gegen eine Anschlagfläche der Beduftungseinrichtung, insbesondere des Hauptkörpers der Beduftungseinrichtung, gedrückt wird.

Gemäß einer bevorzugten Ausführungsform der Erfindung beaufschlagt das Auslöseelement beim Schließen der Kabinentür das Duftmittelbehältnis, vorzugsweise die Abgabeeinrichtung des Duftmittelbehältnisses. Das Auslöseelement wird dann zweckmäßigerweise beim Schließen der Kabinentür von einer Warteposition in eine Auslöseposition überführt und beaufschlagt dabei das Duftmittelbehältnis, vorzugsweise die Abgabeeinrichtung des Duftmittelbehältnisses, derart, dass von der Beduftungseinrichtung ein Duftmittel in die Sanitärkabine abgegeben wird. Beim Öffnen der Kabinentür wird das Auslöseelement bevorzugt von der Auslöseposition in die Warteposition überführt bzw. zurückgestellt. Im Rahmen dieser Ausführungsform wirkt die Kabinentür bevorzugt beim Schließen der Kabinentür kraftbeaufschlagend mit dem Auslöseelement zusammen, sodass die Kraftbeaufschlagung und die Abgabe des Duftmittels, insbesondere die Überführung von der Warteposition in die Auslöseposition bevorzugt gleichzeitig bzw. im Wesentlichen gleichzeitig erfolgen. Im Rahmen dieser Ausführungsform ist der vorzugsweise vorgesehene Gelenkpunkt zwischen dem Auslöseelement und dem Hauptkörper der Beduftungseinrichtung vorzugsweise in einem Endabschnitt, insbesondere am Endpunkt, des Beaufschlagungsabschnittes des Auslöseelementes angeordnet und sehr bevorzugt in Bezug auf die Längserstreckung des Beaufschlagungsabschnittes hinter der Abgabeeinrichtung angeordnet.

Gemäß einer alternativen Ausführungsform der Erfindung beaufschlagt das Auslöseelement beim Öffnen der Kabinentür das Duftmittelbehältnis, vorzugsweise eine Abgabeeinrichtung des Duftmittelbehältnisses. Das Auslöseelement wird dann zweckmäßigerweise beim Öffnen der Kabinentür von einer Warteposition in eine Auslöseposition überführt und beaufschlagt dabei das Duftmittelbehältnis, vorzugsweise die Abgabeeinrichtung des Duftmittelbehältnisses, derart, dass von der Beduftungseinrichtung ein Duftmittel in die Sanitärkabine abgegeben wird. Beim Schließen der Kabinentür wird das Auslöseelement bevorzugt von der Auslöseposition in die Warteposition überführt bzw. zurückgestellt. Im Rahmen dieser Ausführungsform wirkt die Kabinentür bevorzugt beim Schließen der Kabinentür kraftbeaufschlagend mit dem Auslöseelement zusammen, sodass die Kraftbeaufschlagung und die Überführung von der Auslöseposition in die Warteposition insbesondere gleichzeitig erfolgen. Im Rahmen dieser Ausführungsform ist der vorzugsweise vorgesehene Gelenkpunkt zwischen dem Auslöseelement und dem Hauptkörper der Beduftungseinrichtung vorzugsweise in einem Übergangsabschnitt, bevorzugt am Übergangspunkt, zwischen dem Beaufschlagungsabschnitt und dem Zusammenwirkungsabschnitt des Auslöseelementes angeordnet und sehr bevorzugt in Bezug auf die Längserstreckung des Beaufschlagungsabschnittes vor der Abgabeeinrichtung angeordnet.

Es ist sehr bevorzugt, dass die Abgabeeinrichtung eine Feder, insbesondere eine Schraubenfeder, aufweist und dass das Auslöseelement im Zuge der Beaufschlagung des Duftmittelbehältnisses, vorzugsweise der Abgabeeinrichtung, die Feder der Abgabeeinrichtung spannt und dass das Auslöseelement bevorzugt beim Öffnen und/oder Schließen der Kabinentür bevorzugt von der Rückstellkraft der Feder der Abgabeeinrichtung zurückstellbar ist bzw. zurückgestellt wird, bevorzugt von der Auslöseposition in die Warteposition zurückgestellt wird. Zweckmäßigerweise handelt es sich bei dem Duftmittelbehältnis um eine Duftmittelflasche mit einer Abgabeeinrichtung, insbesondere mit einer Sprüheinrichtung, die eine Feder aufweist. Im Zuge der bevorzugten Betätigung des Auslöseelementes zur Abgabe von Duftmittel wird das Duftmittelbehältnis, insbesondere die Abgabeeinrichtung, vorzugsweise die Sprüheinrichtung, beim Schließen und/oder Öffnen der Kabinentür von dem Auslöseelement vorzugsweise heruntergedrückt. Dabei wird zweckmäßigerweise die Feder gespannt. Beim Öffnen und/oder Schließen der Kabinentür stellt die gespannte Feder vorzugsweise die Abgabeeinrichtung, insbesondere die Sprüheinrichtung, zurück und von der Abgabeeinrichtung, insbesondere von der Sprüheinrichtung, wird das Auslöseelement zurückgestellt und zwar ganz besonders bevorzugt von der Auslöseposition in die Warteposition.

Es ist bevorzugt, dass die Beduftungseinrichtung ein Rückstellelement, insbesondere eine Feder, bevorzugt eine Schraubenfeder, aufweist, wobei das Rückstellelement an dem Hauptkörper, insbesondere an der Außenseite des Hauptkörpers, einerseits und an dem Auslöseelement andererseits angeordnet ist und wobei das Rückstellelement bevorzugt beim Schließen der Kabinentür durch Zusammenwirkung des Auslöseelementes mit der Kabinentür gespannt wird und beim Öffnen der Kabinentür, insbesondere durch Zusammenwirkung mit der Kabinentür, entspannt. Es ist im Rahmen dieser Ausführungsform bevorzugt, dass beim Öffnen der Kabinentür das Auslöseelement durch die Rückstellkraft des Rückstellelementes betätigt wird und dabei das Duftmittelbehältnis, vorzugsweise die Abgabeeinrichtung des Duftmittelbehältnisses, derart beaufschlagt, dass von der Beduftungseinrichtung ein Duftmittel in die Sanitärkabine abgebbar ist bzw. abgegeben wird. Vorzugsweise wird das Auslöseelement im Rahmen dieser Ausführungsform beim Schließen der Kabinentür von der Auslöseposition in die Warteposition überführt, insbesondere verschwenkt.

Eine Ausführungsform, der im Rahmen der Erfindung besondere Bedeutung zukommt, ist dadurch gekennzeichnet, dass der Hauptkörper zumindest eine Aufnahmekammer für das Duftmittelbehältnis aufweist. Die Aufnahmekammer wird sehr bevorzugt von einem unteren Teil des Hauptkörpers der Beduftungseinrichtung gebildet. Der oben stehend beschriebene obere Teil des Hauptkörpers und der untere Teil des Hauptkörpers können als einstückiges Aggregat oder auch zwei- bzw. mehrteilig ausgestaltet sein. Das Duftmittelbehältnis ist vorzugsweise vollständig bzw. im Wesentlichen vollständig in der Aufnahmekammer aufnehmbar. Wenn das Duftmittelbehältnis gemäß bevorzugter Ausführungsform als Duftmittelflasche ausgebildet ist, dann ist die Duftmittelflasche bevorzugt im Querschnitt rund, insbesondere kreisrund, ausgebildet und sehr bevorzugt ist dann auch die Aufnahmekammer des Hauptkörpers im Querschnitt rund, insbesondere kreisrund ausgebildet.

Sehr bevorzugt weist der Hauptkörper zumindest eine Aufnahmeöffnung für die Einbringung des Duftmittelbehältnisses in die Aufnahmekammer auf, wobei die Aufnahmeöffnung besonders bevorzugt im eingebrachten Zustand des Duftmittelbehältnisses einem Boden des Duftmittelbehältnisses zugeordnet ist. Grundsätzlich kann die Aufnahmeöffnung im eingebrachten Zustand des Duftmittelbehältnisses aber auch der Abgabeeinrichtung zugeordnet sein. Es ist bevorzugt, dass die Aufnahmeöffnung mit zumindest einem Verschlussdeckel verschließbar ist und dass vorzugsweise ein Bajonettverschluss zwischen der Aufnahmeöffnung und dem Verschlussdeckel vorhanden ist. Grundsätzlich liegen aber auch andere Verbindungsformen zwischen der Aufnahmeöffnung und dem Verschlussdeckel im Rahmen der Erfindung.

Die Aufnahmeöffnung und der Verschlussdeckel bilden bevorzugt einen unteren Abschluss des Hauptkörpers bzw. der Beduftungseinrichtung. Das Duftmittelbehältnis, insbesondere die Duftmittelflasche, kann bevorzugt durch die Aufnahmeöffnung in die Beduftungseinrichtung bzw. in die Aufnahmekammer des Hauptkörpers der Beduftungseinrichtung eingebracht werden und dazu kann besonders bevorzugt zunächst die Abgabeeinrichtung, insbesondere die Sprüheinrichtung, in die Aufnahmekammer eingebracht werden und dann bevorzugt in Richtung des oberen Teils des Hauptkörpers geschoben werden. Zweckmäßigerweise wird die Aufnahmekammer mittels des Verschlussdeckels verschlossen, wenn das Duftmittelbehältnis vollständig in der Aufnahmekammer aufgenommen ist. Dieser Ausführungsform liegt die Erkenntnis zugrunde, dass eine einfache Bestückung der Beduftungseinrichtung mit dem Duftmittelbehältnis möglich ist und dass insbesondere auch ein sehr einfacher Wechsel des Duftmittelbehältnisses erfolgen kann. Insbesondere wenn die Beduftungseinrichtung in einem oberen Teil der Sanitärkabine angeordnet ist, kann auf diese Weise ein einfacher Wechsel des Duftmittelbehältnisses von der - im montierten Zustand der Beduftungseinrichtung insbesondere dem Bodenteil der Sanitärkabine zugeordneten - Unterseite der Beduftungseinrichtung aus erfolgen.

Eine Ausführungsform, der im Rahmen der Erfindung ganz besondere Bedeutung zukommt, ist dadurch gekennzeichnet, dass die Beduftungseinrichtung mit der Maßgabe in der Sanitärkabine angeordnet ist, insbesondere mit der Maßgabe an der die Kabinentür aufweisenden Kabinenseite angeordnet ist, dass das Auslöseelement, vorzugsweise der Auslösebügel, bei geöffneter Kabinentür in die Türöffnung der Sanitärkabine hineinragt und beim Schließen der Kabinentür, bevorzugt von der Kabinentür, aus der Türöffnung herausgedrückt wird und dabei insbesondere von der Warteposition in die Auslöseposition oder von der Auslöseposition in die Warteposition überführt, insbesondere verschwenkt, wird. Auf diese Weise kann die bevorzugte Zusammenwirkung von der Kabinentür und dem Auslöseelement besonders funktionssicher realisiert werden. Zweckmäßigerweise ragt das Auslöseelement in der Warteposition oder in der Auslöseposition bei geöffneter Kabinentür in die Türöffnung hinein. Beim Schließen der Kabinentür wird das Auslöseelement sehr bevorzugt aus der Türöffnung herausgedrückt und zwar ganz besonders bevorzugt von der Kabinentür und weiter bevorzugt durch Kontakt, insbesondere direkten Kontakt, mit der Kabinentür, vorzugsweise mit der Innenseite der Kabinentür. Dabei wird das Auslöseelement vorzugsweise von der Warteposition in die Auslöseposition oder von der Auslöseposition in die Warteposition überführt, insbesondere verschwenkt. Die Kraft zur Überführung des Auslöseelementes von der Warteposition in die Auslöseposition oder von der Auslöseposition in die Warteposition wird im Rahmen dieser besonders bevorzugten Ausführungsform durch das Schließen der Kabinentür von dem Benutzer aufgebracht.

Es ist im Rahmen der Erfindung bevorzugt, dass die Sanitärkabine zumindest drei, vorzugsweise zumindest vier, Eckpfosten aufweist, die das Bodenteil mit dem Deckenteil verbinden. Vorzugsweise sind auch zumindest zwei Seitenwandteile der Sanitärkabine über zumindest einen Eckpfosten miteinander verbunden. Empfohlenermaßen besitzt die Sanitärkabine somit ein Deckenteil, ein Bodenteil und vier Eckpfosten, die das Deckenteil mit dem Bodenteil verbinden. Weiter bevorzugt weist die Sanitärkabine zumindest drei, insbesondere drei Seitenwandteile und zumindest eine verschwenkbare Kabinentür auf. Vorzugsweise verbindet ein Seitenwandteil der Sanitärkabine zwei Eckpfosten miteinander. Darüber hinaus verbindet gemäß bevorzugter Ausführungsform auch die verschwenkbare Kabinentür im geschlossenen Zustand zwei Eckpfosten miteinander. Die Kabinentür bildet somit vorzugsweise ein viertes Wandteil der Sanitärkabine. An der Kabinentür ist vorzugsweise zumindest ein Türgriff und/oder zumindest ein Verriegelungselement angeordnet. Gemäß sehr bevorzugter Ausführungsform ist die Sanitärkabine vierseitig ausgebildet und drei Seiten der Kabine werden von einem jeweils zwei Eckpfosten miteinander verbindenden Seitenwandteil gebildet und/oder eine vierte Seite ist so ausgestaltet, dass zwischen zwei Eckpfosten die verschwenkbare Kabinentür angeordnet ist.

Bevorzugt ist eine die Schwenkachse S der verschwenkbaren Kabinentür bildende Türstange neben, insbesondere unmittelbar neben, einem Eckpfosten der Sanitärkabine angeordnet. Die Türstange erstreckt sich vorzugsweise von dem Bodenteil bis zu dem Deckenteil der Kabine und greift bevorzugt jeweils in ein Aufnahmeelement des Bodenteils und des Deckenteils ein. Die Kabinentür ist insbesondere um die Türstange verschwenkbar und somit vorzugsweise schwenkbeweglich an der Türstange angeordnet. Gemäß einer weiteren bevorzugten Ausführungsform ist die verschwenkbare Kabinentür an einem der Eckpfosten angelenkt und sehr bevorzugt über Scharniere angelenkt. Bei beiden Ausgestaltungen ist an dem anderen der beiden Eckpfosten weiter bevorzugt eine Anschlagleiste für die Kabinentür vorhanden und hier lässt sich empfohlenermaßen die Kabinentür über zumindest ein Kabinenschloss verschließen.

Gemäß bevorzugter Ausführungsform der Erfindung ist die Beduftungseinrichtung an einem der Eckpfosten angeordnet und sehr bevorzugt an einem der Schwenkachse S der Kabinentür zugeordneten Eckpfosten angeordnet.

Dass der Eckpfosten, an dem die Beduftungseinrichtung vorzugsweise angeordnet ist, der Schwenkachse S der Kabinentür zugeordnet ist, meint insbesondere, dass sich die Schwenkachse S der Kabinentür neben, insbesondere unmittelbar neben, diesem Eckpfosten befindet. Es ist bevorzugt, dass die Beduftungseinrichtung im oberen Drittel, vorzugsweise im oberen Viertel, der Sanitärkabine - insbesondere im oberen Drittel, vorzugsweise im oberen Viertel, des Eckpfostens, an dem die Beduftungseinrichtung vorgesehen ist - angeordnet ist. Dieser Ausführungsform liegt die Erkenntnis zugrunde, dass die Beduftungseinrichtung sich dann in etwa auf der Höhe des Gesichtes, insbesondere der Nase, eines Benutzers befindet, sodass der Benutzer das in die Kabine abgegebene Duftmittel besonders rasch wahrnimmt.

Bevorzugt erfolgt die Verbindung zwischen der Beduftungseinrichtung und der Sanitärkabine, vorzugsweise zwischen der Beduftungseinrichtung und dem Eckpfosten, mittels zumindest eines Schraubelementes, das insbesondere den Hauptkörper bzw. eine Wandung des Hauptkörpers der Beduftungseinrichtung durchsetzt und mit der Sanitärkabine, vorzugsweise mit dem Eckpfosten, verschraubt wird. Vorzugsweise sind zumindest zwei, bevorzugt zumindest drei solcher Schraubelemente vorgesehen. Die Schraubelemente durchsetzen zweckmäßigerweise den unteren Teil des Hauptkörpers bzw. eine Wandung des unteren Teils des Hauptkörpers der Beduftungseinrichtung, sodass vorzugsweise dieser untere Teil des Hauptkörpers der Beduftungseinrichtung mit der Sanitärkabine, vorzugsweise mit dem Eckpfosten, verschraubt wird. Gemäß einer bevorzugten Ausführungsform wird der Hauptkörper der Beduftungseinrichtung direkt mit der Sanitärkabine, vorzugsweise mit dem Eckpfosten, verschraubt. Gemäß einer weiteren Ausführungsform der Erfindung ist ein Zwischenelement, insbesondere ein Zwischenblech, zwischen dem Hauptkörper der Beduftungseinrichtung und der Sanitärkabine, insbesondere dem Eckpfosten, angeordnet. Das Zwischenelement dient insbesondere dazu, dass die Beduftungseinrichtung im Hinblick auf die Zusammenwirkung mit der Tür, insbesondere im Hinblick auf das Hineinragen des Auslöseelementes in die Türöffnung, funktionssicher ausgerichtet ist bzw. ausgerichtet werden kann. Gemäß sehr bevorzugter Ausführungsform der Erfindung ist die Beduftungseinrichtung somit an einem der Schwenkachse S der Kabinentür zugeordneten Eckpfosten der Sanitärkabine angeordnet und insbesondere mit diesem Eckpfosten verschraubt.

Gemäß einer alternativen Ausführungsform der erfindungsgemäßen Sanitärkabine ist die Beduftungseinrichtung an einem Türrahmen der Kabinentür angeordnet. Diese Ausführungsform ist vor allem dann von besonderer Bedeutung, wenn beispielsweise die vierte Seite einer vierseitigen Kabine nicht vollständig von einer Kabinentür gebildet wird, sondern wenn eine vierte Seitenwand vorgesehen ist, die die Türöffnung für die Kabinentür aufweist. Dann ist die Kabinentür zweckmäßigerweise von einem Türrahmen umgeben und vorzugsweise an dem Türrahmen angelenkt und an der kabineninnenseitigen Seite dieses Türrahmens ist bevorzugt die Beduftungseinrichtung angeordnet und zwar sehr bevorzugt an der der Schwenkachse S der Kabinentür zugeordneten Seite des Türrahmens.

Es ist somit besonders bevorzugt, dass die zumindest eine Beduftungseinrichtung an einem Eckpfosten der Sanitärkabine, insbesondere an einem der Schwenkachse S der Kabinentür zugeordneten Eckpfosten der Sanitärkabine und/oder an einem Türrahmen der Kabinentür angeordnet ist.

Eine bevorzugte Ausführungsform der Erfindung ist dadurch gekennzeichnet, dass in der Sanitärkabine zumindest ein Fäkalientank und/oder zumindest eine Sanitäreinrichtung, insbesondere zumindest ein WC-Becken und/oder zumindest ein Urinal und/oder zumindest ein Handwaschbecken, angeordnet ist/sind. Es hat sich bewährt, dass die Sanitärkabine zumindest ein Frischwasserreservoir aufweist, das in Verbindung bzw. Fluidverbindung mit der zumindest einen Sanitäreinrichtung, insbesondere mit dem Handwaschbecken und/oder mit dem WC-Becken und/oder mit dem Urinal, steht. Das Frischwasser kann in dem Handwaschbecken von einem Benutzer zur Reinigung der Hände verwendet werden und/oder es kann als Spülflüssigkeit in dem Urinal und/oder in dem WC-Becken zum Einsatz kommen. Es ist möglich, dass zumindest ein Eckpfosten der Sanitärkabine bereichsweise innen hohl ausgebildet ist und dass das zumindest eine Frischwasserreservoir in diesem Hohlraum angeordnet ist. Grundsätzlich kann das Frischwasserreservoir aber auch an anderen Stellen der Sanitärkabine angeordnet sein.

Vorzugsweise ist die Sanitärkabine zumindest teilweise, bevorzugt vollständig bzw. im Wesentlichen vollständig, aus zumindest einem Kunststoff, bevorzugt aus zumindest einem thermoplastischen Kunststoff, sehr bevorzugt aus Polypropylen und/oder Polyethylen, ausgebildet. Bei dem thermoplastischen Kunststoff handelt es sich somit bevorzugt um Polypropylen und/oder um Polyethylen. Bevorzugt handelt es sich bei dem thermoplastischen Kunststoff um Polyethylen. Es ist weiter bevorzugt, dass zumindest das Bodenteil und/oder das Deckenteil und/oder die Seitenwandteile und/oder die Eckpfosten und/oder die Kabinentür und/oder die zumindest eine Sanitäreinrichtung und/oder die zumindest eine Beduftungseinrichtung aus dem zumindest einen thermoplastischen Kunststoff ausgebildet ist/sind, sehr bevorzugt aus Polyethylen ausgebildet ist/sind und besonders bevorzugt aus Polyethylen besteht/bestehen bzw. im Wesentlichen besteht/bestehen. Es ist sehr bevorzugt, dass diese vorstehend aufgeführten Komponenten jeweils im Rotationsverfahren bzw. durch Rotationsformen gefertigt sind. Dass eine Komponente der Sanitärkabine aus einem thermoplastischen Kunststoff, bevorzugt aus Polyethylen, besteht bzw. im Wesentlichen besteht, meint im Rahmen der Erfindung insbesondere, dass dem Kunststoff Additive bzw. Zuschläge zugesetzt sein können. Die Komponente besteht dann vorzugsweise zu mindestens 95 Gew.-%, bevorzugt zu mindestens 98 Gew.-% aus dem zumindest einem Kunststoff bzw. thermoplastischen Kunststoff. Es ist bevorzugt, dass das Bodenteil und/oder das Deckenteil und/oder die Seitenwandteile und/oder die Eckpfosten und/oder die Kabinentür und/oder die zumindest eine Sanitäreinrichtung als einstückige Kunststoffteile ausgebildet sind.

Zur Lösung des technischen Problems lehrt die Erfindung außerdem ein Verfahren zum Betrieb einer oben stehend beschriebenen Sanitärkabine, insbesondere einer Toilettenkabine, wobei beim Schließen und/oder beim Öffnen der verschwenkbaren Kabinentür das Auslöseelement, insbesondere der Auslösebügel, der Beduftungseinrichtung das Duftmittelbehältnis, vorzugsweise eine Abgabeeinrichtung des Duftmittelbehältnisses, derart beaufschlagt, dass von der Beduftungseinrichtung ein Duftmittel in die Sanitärkabine abgegeben wird, und wobei dazu bevorzugt das Auslöseelement durch Zusammenwirkung mit der Kabinentür betätigt wird.

Gemäß bevorzugter Ausführungsform wird somit beim Schließen und/oder Öffnen der verschwenkbaren Kabinentür das Auslöseelement, insbesondere der Auslösebügel, der Beduftungseinrichtung durch Zusammenwirkung mit der Kabinentür betätigt und das Auslöseelement beaufschlagt dabei das Duftmittelbehältnis, vorzugsweise eine Abgabeeinrichtung des Duftmittelbehältnisses, derart, dass von der Beduftungseinrichtung ein Duftmittel in die Sanitärkabine abgegeben wird.

Der Erfindung liegt die Erkenntnis zugrunde, dass bei der erfindungsgemäßen Sanitärkabine ein angenehmer Geruch im Innenraum funktionssicher durch das Duftmittel bzw. angenehm riechende Duftmittel realisiert werden kann. Insbesondere können störende Gerüche in der erfindungsgemäßen Sanitärkabine effektiv und funktionssicher durch die im Innenraum der Sanitärkabine angeordnete Beduftungseinrichtung vermieden werden. Die Beduftungseinrichtung zeichnet sich insbesondere durch ihre Funktionssicherheit und durch ihre Einfachheit aus. Die Beduftungseinrichtung gibt insbesondere lediglich dann Duftmittel in die Sanitärkabine ab, wenn die Sanitärkabine auch tatsächlich benutzt wird, sodass das Duftmittel sehr effizient eingesetzt wird. Wenn gemäß bevorzugter Ausführungsform ein Auffangabschnitt und/oder ein Speicherelement für das Duftmittel vorhanden ist, wird darüber hinaus über einen längeren Zeitraum Duftmittel in den Innenraum der Sanitärkabine abgegeben. Es ist außerdem zu betonen, dass die bevorzugte Betätigung des Auslöseelementes der erfindungsgemäßen Beduftungseinrichtung ohne aufwendige Maßnahmen und insbesondere durch Zusammenwirkung mit der Kabinentür erfolgt. Durch die bevorzugte Anordnung der Beduftungseinrichtung in Türnähe und gemäß besonders bevorzugter Ausführungsform mit einem in die Türöffnung hineinragenden Auslöseelement, kann ohne aufwendige Ausgestaltung eine funktionssichere Betätigung des Auslöseelementes erfolgen. Die einfache und funktionssichere Ausgestaltung der Beduftungseinrichtung führt insbesondere auch zu einer vorteilhaften Wirtschaftlichkeit der Beduftungseinrichtung. Darüber hinaus ist es mit der erfindungsgemäßen Beduftungseinrichtung möglich, das Duftmittelbehältnis mit wenig Aufwand zu wechseln und/oder nachzufüllen. Das kann beispielsweise im Rahmen einer Zwischenreinigung oder Endreinigung der Sanitärkabine geschehen.

Nachfolgend wird die Erfindung anhand einer lediglich ein Ausführungsbeispiel darstellenden Zeichnung näher erläutert. Es zeigen in schematischer Darstellung:
- Fig. 1: eine perspektivische Ansicht einer erfindungsgemäßen Sanitärkabine bzw. Toilettenkabine in einer ersten bevorzugten Ausführungsform
- Fig. 2: eine Innenansicht des Gegenstandes gemäß Fig. 1 mit einer erfindungsgemäßen Beduftungseinrichtung in der Warteposition
- Fig. 3: eine Innenansicht des Gegenstandes gemäß Fig. 1 mit einer erfindungsgemäßen Beduftungseinrichtung in der Auslöseposition
- Fig. 4: eine Explosionsdarstellung einer erfindungsgemäßen Beduftungseinrichtung
- Fig. 5: eine Seitenansicht einer erfindungsgemäßen Beduftungseinrichtung in der Warteposition
- Fig. 6: eine weitere Seitenansicht einer erfindungsgemäßen Beduftungseinrichtung in der Auslöseposition
- Fig. 7: eine perspektivische Ansicht einer erfindungsgemäßen Beduftungseinrichtung in der Auslöseposition
- Fig. 8: eine perspektivische Ansicht einer erfindungsgemäßen Sanitärkabine bzw. Toilettenkabine in einer zweiten bevorzugten Ausführungsform
- Fig. 9: einen Horizontalschnitt durch den Gegenstand gemäß Fig. 8 im Bereich der Schwenkachse der Kabinentür.

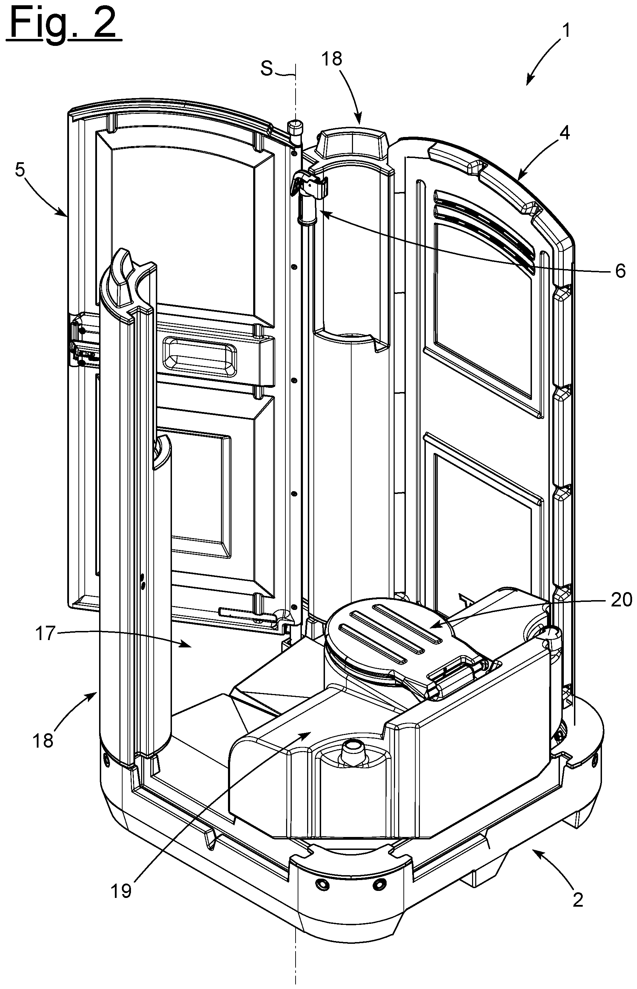

Die Figuren zeigen eine erfindungsgemäße Sanitärkabine, die bevorzugt und im Ausführungsbeispiel als Toilettenkabine 1 ausgebildet ist. Zweckmäßigerweise und im Ausführungsbeispiel gemäß den Figuren handelt es sich um eine mobile bzw. transportable Toilettenkabine 1. Die Toilettenkabine 1 weist bevorzugt und im Ausführungsbeispiel ein Bodenteil 2, ein Deckenteil 3, drei Seitenwandteile 4, eine verschwenkbare Kabinentür 5 und vier Eckpfosten 18 auf. Im Ausführungsbeispiel wird das Bodenteil 2 mit dem Deckenteil 3 über die vier Eckpfosten 18 verbunden und außerdem werden Bodenteil 2 und Deckenteil 3 über die drei Seitenwandteile 4 sowie über die Kabinentür 5 miteinander verbunden. Die Toilettenkabine 1 weist bevorzugt und im Ausführungsbeispiel eine Türöffnung 17 auf, über die die Toilettenkabine 1 betreten werden kann. Im Ausführungsbeispiel und bevorzugt weist die Toilettenkabine 1 außerdem einen Fäkalientank 19 auf. Das ist insbesondere in den Figuren 2, 3 und 8 zu erkennen. Darüber hinaus ist in der Toilettenkabine 1 bevorzugt zumindest eine Sanitäreinrichtung, vorzugsweise und im Ausführungsbeispiel ein WC-Becken 20 angeordnet. Das WC-Becken 20 ist zweckmäßigerweise und im Ausführungsbeispiel oberhalb des Fäkalientanks 19 angeordnet. Die Fäkalien und/oder das Abwasser aus dem WC-Becken 20 werden bevorzugt und im Ausführungsbeispiel direkt in dem Fäkalientank 19 aufgenommen.

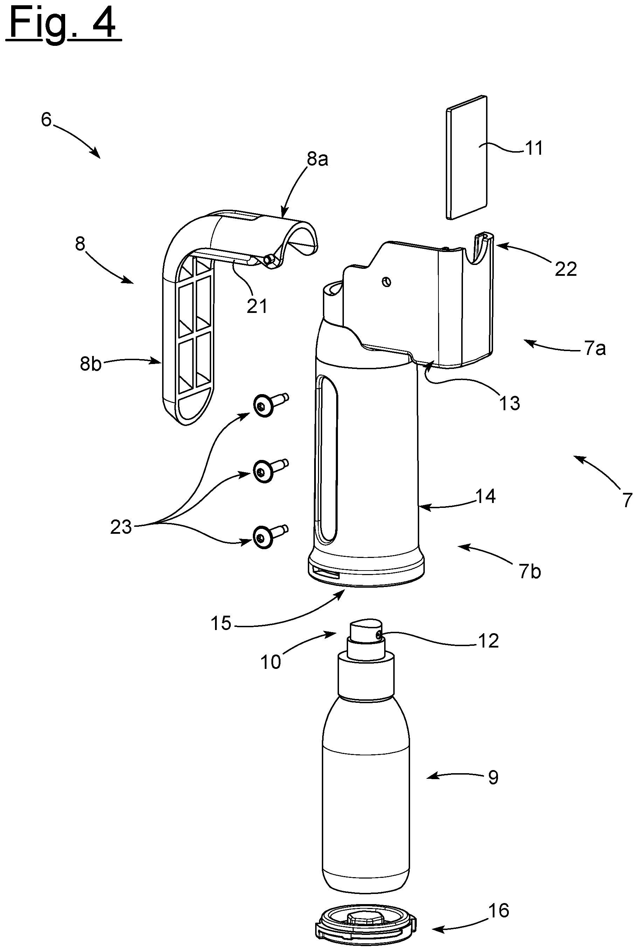

Im Innenraum der Toilettenkabine 1 ist im Ausführungsbeispiel eine Beduftungseinrichtung 6 angeordnet. Die Beduftungseinrichtung 6 weist erfindungsgemäß und im Ausführungsbeispiel einen Hauptkörper 7, zumindest ein - vorzugsweise und im Ausführungsbeispiel als Auslösebügel 8 ausgestaltetes - Auslöseelement und zumindest ein Duftmittelbehältnis 9 auf (Fig. 4). Die Beduftungseinrichtung 6 ist vorzugsweise und im Ausführungsbeispiel gemäß den Figuren mit der Maßgabe im Innenraum der Toilettenkabine 1 angeordnet, dass der Auslösebügel 8 beim Schließen der Kabinentür 5 durch Zusammenwirkung mit der Kabinentür 5 - vorzugsweise und im Ausführungsbeispiel durch Zusammenwirkung mit der Innenseite der Kabinentür 5 - betätigbar ist bzw. betätigt wird. Dabei beaufschlagt der Auslösebügel 8 bevorzugt und im Ausführungsbeispiel eine als Sprühkopf 10 ausgebildete Abgabeeinrichtung des Duftmittelbehältnisses 9 derart, dass von der Beduftungseinrichtung 6 ein Duftmittel in die Toilettenkabine 1 sprühbar ist bzw. gesprüht wird.

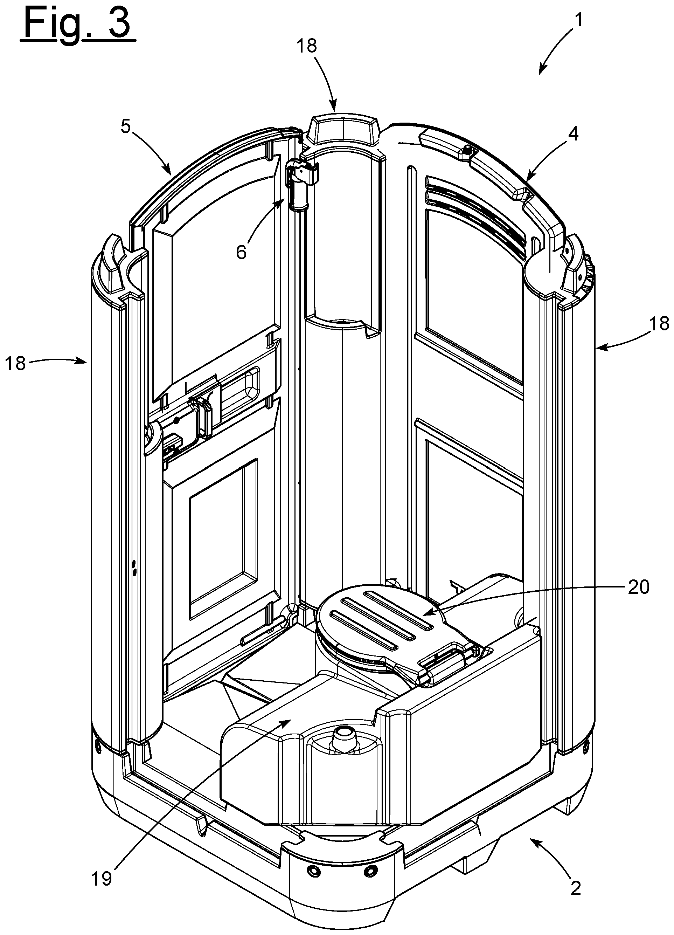

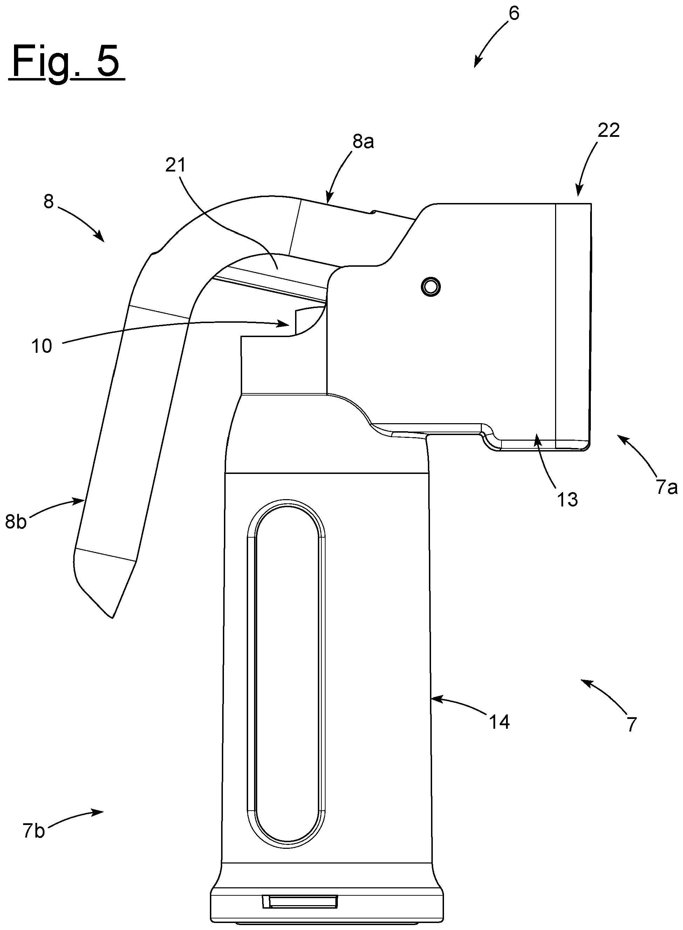

Der Auslösebügel 8 ist bevorzugt und im Ausführungsbeispiel gemäß den Figuren an dem Hauptkörper 7 der Beduftungseinrichtung 6 angelenkt. Das ist etwa in den Figuren 5, 6 und 7 zu erkennen. Beim Schließen der Kabinentür 5 wird der Auslösebügel 8 bevorzugt und im Ausführungsbeispiel gemäß den Figuren durch Zusammenwirkung mit der Kabinentür 5 von einer Warteposition in eine Auslöseposition verschwenkt. Die Warteposition ist beispielsweise in den Figuren 2 und 5 dargestellt. Die Auslöseposition ist etwa in den Figuren 3, 6 und 7 dargestellt. Sehr bevorzugt und im Ausführungsbeispiel gemäß den Figuren beaufschlagt der Auslösebügel 8 bei der Verschwenkung von der Warteposition in die Auslöseposition den Sprühkopf 10 des Duftmittelbehältnisses 9. Bevorzugt und im Ausführungsbeispiel erfolgt die Verschwenkung des Auslösebügels 8 von der Warteposition in die Auslöseposition durch direkten Kontakt der Innenseite der Kabinentür 5 mit dem Auslösebügel 8 beim Schließen der Kabinentür 5 (Figuren 2 und 3). Der Auslösebügel 8 weist bevorzugt und im Ausführungsbeispiel einen Beaufschlagungsabschnitt 8a für die Beaufschlagung des Sprühkopfes 10 des Duftmittelbehältnisses 9 und einen Zusammenwirkungsabschnitt 8b für die Zusammenwirkung mit der Kabinentür 5 auf. Sehr bevorzugt und im Ausführungsbeispiel sind der Beaufschlagungsabschnitt 8a und der Zusammenwirkungsabschnitt 8b im rechten Winkel bzw. im Wesentlichen im rechten Winkel zueinander angeordnet. Der Gelenkpunkt zwischen dem Hauptkörper 7 der Beduftungseinrichtung 6 und dem Auslösebügel 8 ist vorzugsweise und im Ausführungsbeispiel im Endabschnitt bzw. am Endpunkt des Beaufschlagungsabschnittes 8a angeordnet. Weiter bevorzugt und im Ausführungsbeispiel ist der Gelenkpunkt in Bezug auf die Längserstreckung des Beaufschlagungsabschnittes 8a hinter der Abgabeeinrichtung bzw. hinter dem Sprühkopf 10 angeordnet.

Für die Beaufschlagung des Sprühkopfes 10 weist der Auslösebügel 8 bzw. der Beaufschlagungsabschnitt 8a des Auslösebügels 8 vorzugsweise und im Ausführungsbeispiel einen Beaufschlagungsfortsatz 21 auf. Die Beaufschlagung des Sprühkopfes 10 des Duftmittelbehältnisses 9 erfolgt zweckmäßigerweise und im Ausführungsbeispiel im Zuge der Verschwenkung des Auslösebügels 8 von der Warteposition in die Auslöseposition. Bevorzugt und im Ausführungsbeispiel handelt es sich bei dem Duftmittelbehältnis 9 um eine Duftmittelflasche mit einer insbesondere als Sprühkopf 10 ausgebildeten Abgabeeinrichtung. Der Sprühkopf 10 wird vorzugsweise und im Ausführungsbeispiel beim Schließen der Kabinentür von dem Auslösebügel 8 heruntergedrückt und dabei wird aus einer Düse 12 des Sprühkopfes 10 Duftmittel in die Toilettenkabine 1 gesprüht.

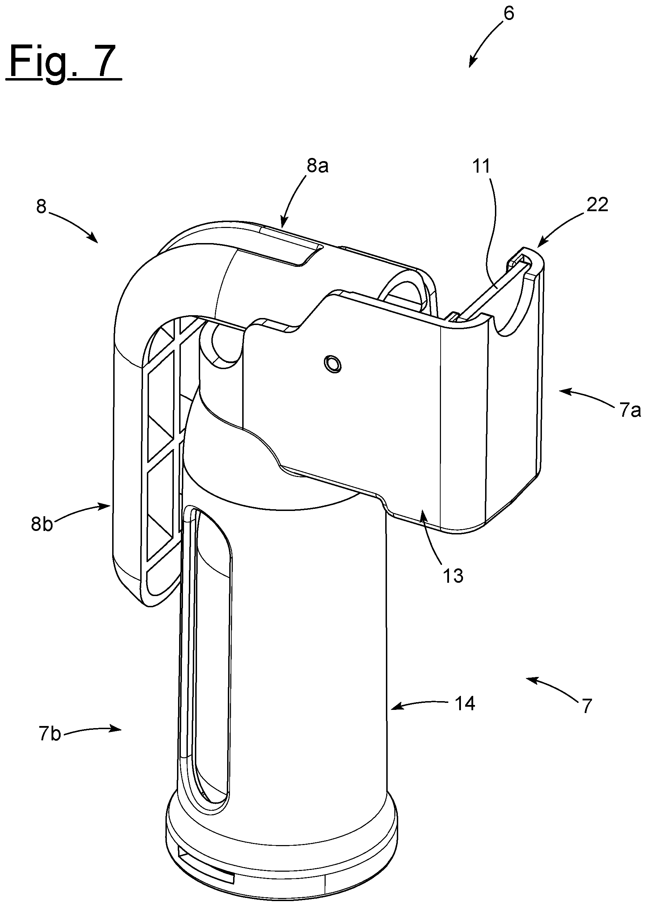

Gemäß sehr bevorzugter Ausführungsform der Erfindung und im Ausführungsbeispiel gemäß den Figuren weist die Beduftungseinrichtung 6 ein Speicherelement in Form eines Speicherfilzes 11 auf, wobei Duftmittel, das aus dem Sprühkopf 10 abgegeben wird, in dem Speicherfilz 11 speicherbar ist bzw. gespeichert wird (z. B. Figuren 4 und 7). Bevorzugt und im Ausführungsbeispiel ist der Speicherfilz 11 dem Sprühkopf 10 bzw. der Düse 12 des Sprühkopfes 10 gegenüberliegend angeordnet, sodass der Sprühkopf 10 bevorzugt und im Ausführungsbeispiel zumindest einen Teil des Duftmittels gegen den Speicherfilz 11 sprüht. Der Hauptkörper 7 der Beduftungseinrichtung 6 weist bevorzugt und im Ausführungsbeispiel eine Speicherelementaufnahme 22 für die Aufnahme des Speicherfilzes 11 auf. Der Speicherfilz 11 kann vorzugsweise und im Ausführungsbeispiel in die Speicherelementaufnahme 22 eingeschoben werden (Figuren 4 und 7). Sehr bevorzugt und im Ausführungsbeispiel ist der Speicherfilz 11 bezüglich seiner flächigen Erstreckung vertikal bzw. im Wesentlichen vertikal in der Beduftungseinrichtung 6, insbesondere in der Speicherelementaufnahme 22, angeordnet bzw. aufgenommen (Figuren 4 und 7). Bevorzugt und im Ausführungsbeispiel gemäß den Figuren ist der Speicherfilz 11 beabstandet von dem Sprühkopf 10, insbesondere von der Düse 12 des Sprühkopfes 10 angeordnet. Das gilt vorzugsweise auch für die Speicherelementaufnahme 22.

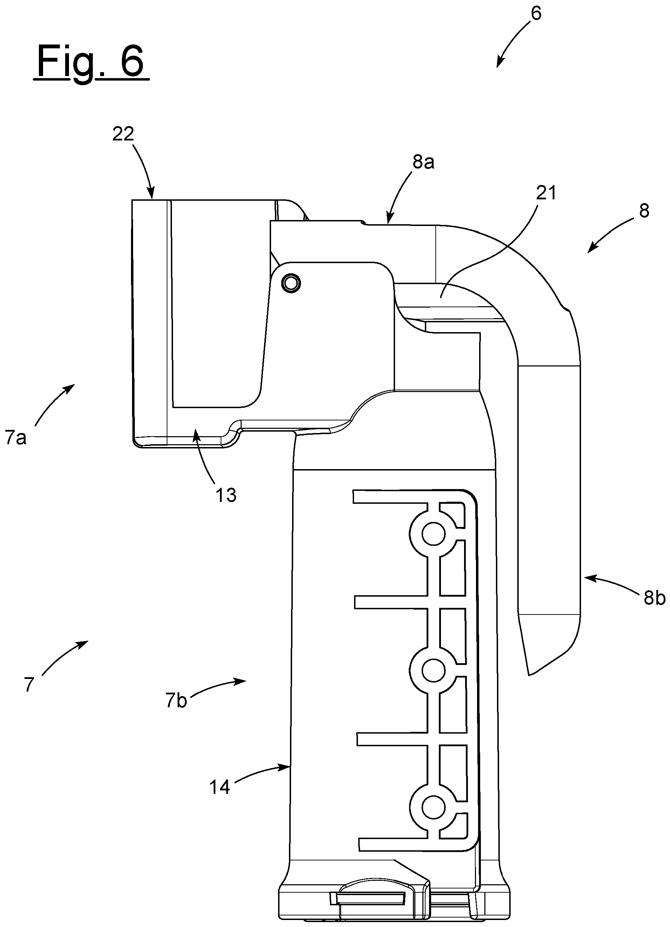

Der Hauptkörper 7 der Beduftungseinrichtung 6 weist bevorzugt und im Ausführungsbeispiel gemäß den Figuren ein Auffangreservoir 13 auf, in dem von dem Sprühkopf 10 abgegebenes Duftmittel auffangbar ist bzw. aufgefangen wird. Das Auffangreservoir 13 ist bevorzugt und im Ausführungsbeispiel in Duftmittelsprührichtung zwischen dem Sprühkopf 10 und dem Speicherfilz 11 angeordnet (Figuren 4 bis 7). Der Hauptkörper 7 der Beduftungseinrichtung 6 weist im Übrigen bevorzugt und im Ausführungsbeispiel einen oberen Teil 7a und einen unteren Teil 7b auf. Der obere Teil 7a des Hauptkörpers 7 umgibt bevorzugt und im Ausführungsbeispiel zumindest einen Abschnitt der Sprüheinrichtung 10 und zweckmäßigerweise und im Ausführungsbeispiel ist in Duftmittelsprührichtung zunächst das Auffangreservoir 13 und anschließend die Speicherelementaufnahme 22 angeordnet (z. B. Figur 6). Der Auslösebügel 8 ist vorzugsweise und im Ausführungsbeispiel an dem oberen Teil 7a des Hauptkörpers 7 angelenkt.

Sehr bevorzugt und im Ausführungsbeispiel gemäß den Figuren ist der Auslösebügel 8 derart an dem Hauptkörper 7 - vorzugsweise und im Ausführungsbeispiel an dem oberen Teil 7a des Hauptkörpers 7 - angelenkt, dass er den Sprühkopf 10 übergreift. Vorzugsweise und im Ausführungsbeispiel erfolgt die Beaufschlagung des Sprühkopfes 10 mittels des Beaufschlagungsfortsatzes 21 des Auslösebügels 8. Im Zuge der Betätigung des Auslösebügels 8 drückt der Auslösebügel 8 bzw. der Beaufschlagungsfortsatz 21 des Auslösebügels 8 vorzugsweise und im Ausführungsbeispiel die als Sprühkopf 10 ausgebildete Sprüheinrichtung zur Abgabe eines Duftmittels, insbesondere zur Abgabe eines Sprühstoßes eines Duftmittels, herunter.

Gemäß besonders bevorzugter Ausführungsform der erfindungsgemäßen Sanitärkabine bzw. Toilettenkabine 1 und im Ausführungsbeispiel weist der Sprühkopf 10 eine Feder, insbesondere eine Schraubenfeder, auf. Die Feder ist in den Figuren nicht näher dargestellt. Der Auslösebügel 8 spannt diese Feder des Sprühkopfes 10 vorzugsweise bei der Beaufschlagung des Sprühkopfes 10 im Zuge des Schließens der Kabinentür 5. Beim Öffnen der Kabinentür 5 ist der Auslösebügel 8 bevorzugt und im Ausführungsbeispiel von der Rückstellkraft der Feder des Sprühkopfes 10 zurückstellbar bzw. wird zurückgestellt und zwar insbesondere und im Ausführungsbeispiel gemäß den Figuren von der Auslöseposition in die Warteposition. Zweckmäßigerweise und im Ausführungsbeispiel handelt es sich bei dem Duftmittelbehältnis 9 um eine Duftmittelflasche mit einer als Sprühkopf 10 ausgebildeten Sprüheinrichtung, wobei dieser Sprühkopf 10 vorzugsweise die Feder aufweist. Im Zuge der Betätigung des Auslösebügels 8 zur Abgabe von Duftmittel wird die der Sprühkopf 10 bevorzugt und im Ausführungsbeispiel beim Schließen der Kabinentür 5 von dem Auslösebügel 8 heruntergedrückt. Dabei wird zweckmäßigerweise und im Ausführungsbeispiel die Feder gespannt. Beim Öffnen der Kabinentür 5 stellt die gespannte Feder den Sprühkopf 10 vorzugsweise zurück und bevorzugt und im Ausführungsbeispiel wird von dem Sprühkopf 10 auch der Auslösebügel 8 zurückgestellt und zwar ganz besonders bevorzugt von der Auslöseposition in Warteposition.

Gemäß einer weiteren sehr bevorzugten Ausführungsform der Erfindung weist der Hauptkörper 7 eine Aufnahmekammer 14 für das Duftmittelbehältnis 9 auf (Figuren 4 bis 7). Die Aufnahmekammer 14 wird sehr bevorzugt von dem unteren Teil 7b des Hauptkörpers 7 der Beduftungseinrichtung 6 gebildet. Das Duftmittelbehältnis 9 ist vorzugsweise und im Ausführungsbeispiel vollständig in der Aufnahmekammer 14 aufnehmbar. Sehr bevorzugt und im Ausführungsbeispiel gemäß den Figuren ist sowohl das als Duftmittelflasche ausgebildete Duftmittelbehältnis 9 als auch die Aufnahmekammer 14 im Querschnitt rund, insbesondere kreisrund ausgebildet.

Der Hauptkörper 7 bzw. der untere Teil 7b des Hauptkörpers weist sehr bevorzugt und im Ausführungsbeispiel eine Aufnahmeöffnung 15 für die Einbringung des Duftmittelbehältnisses 9 in die Aufnahmekammer 14 auf, wobei die Aufnahmeöffnung 15 sehr bevorzugt und im Ausführungsbeispiel gemäß den Figuren im eingebrachten Zustand des Duftmittelbehältnisses 9 dem Boden des Duftmittelbehältnisses 9 zugeordnet ist. Gemäß sehr bevorzugter Ausführungsform und im Ausführungsbeispiel ist die Aufnahmeöffnung 15 mit einem Verschlussdeckel 16 verschließbar und weiter bevorzugt und im Ausführungsbeispiel ist zwischen der Aufnahmeöffnung 15 und dem Verschlussdeckel 16 ein Bajonettverschluss vorgesehen. Das ist insbesondere in der Figur 4 zu erkennen.

Es ist im Rahmen der Erfindung ganz besonders bevorzugt, dass die Beduftungseinrichtung 6 mit der Maßgabe in der Sanitärkabine bzw. Toilettenkabine 1 angeordnet ist, insbesondere mit der Maßgabe an der die Kabinentür 5 aufweisenden Kabinenseite angeordnet ist, dass der Auslösebügel 8 bei geöffneter Kabinentür 5 in die Türöffnung 17 der Toilettenkabine 1 hineinragt (Figur 2) und beim Schließen der Kabinentür 5, bevorzugt von der Kabinentür 5, aus der Türöffnung 17 herausgedrückt wird (Figur 3). Auf diese Weise kann die Zusammenwirkung von der Kabinentür 5 und dem Auslösebügel 8 besonders funktionssicher realisiert werden. Zweckmäßigerweise und im Ausführungsbeispiel ragt der Auslösebügel 8 in der Warteposition bei geöffneter Kabinentür 5 in die Türöffnung 17 hinein. Beim Schließen der Kabinentür 5 wird der Auslösebügel 8 sehr bevorzugt und im Ausführungsbeispiel aus der Türöffnung 17 herausgedrückt und zwar ganz besonders bevorzugt von der Kabinentür 5 und weiter bevorzugt und im Ausführungsbeispiel durch direkten Kontakt mit der Kabinentür 5 bzw. mit der Innenseite der Kabinentür 5. Dabei wird insbesondere und im Ausführungsbeispiel der Auslösebügel 8 von der Warteposition in die Auslöseposition verschwenkt. Die Kraft zur Überführung bzw. Verschwenkung und auch für die Beaufschlagung des Sprühkopfes 10 zur Abgabe von Duftmittel wird im Rahmen dieser bevorzugten Ausführungsform und im Ausführungsbeispiel durch das Schließen der Kabinentür 5 von dem Benutzer aufgebracht.

Gemäß sehr bevorzugter Ausführungsform der erfindungsgemäßen Sanitärkabine bzw. Toilettenkabine 1 ist die Toilettenkabine 1 vierseitig ausgebildet und drei Seiten der Toilettenkabine 1 werden von einem jeweils zwei Eckpfosten 18 miteinander verbindenden Seitenwandteil 4 gebildet und eine vierte Seite ist so ausgestaltet, dass zwischen zwei Eckpfosten 18 die verschwenkbare Kabinentür 5 angeordnet ist. Die verschwenkbare Kabinentür 5 ist mit ihrer Schwenkachse S sehr bevorzugt und im Ausführungsbeispiel neben, insbesondere unmittelbar neben, einem der Eckpfosten 18 angeordnet (z. B. Figur 2). An dem anderen der beiden Eckpfosten 18 ist weiter bevorzugt und im Ausführungsbeispiel eine Anschlagsleiste für die Kabinentür 5 vorhanden.

Gemäß sehr bevorzugter Ausführungsform der Erfindung und im Ausführungsbeispiel gemäß den Figuren ist die Beduftungseinrichtung 6 an dem der Schwenkachse S der Kabinentür 5 zugeordneten Eckpfosten 18 angeordnet. Gemäß besonders bevorzugter Ausführungsform und im Ausführungsbeispiel ist die Beduftungseinrichtung 6 im oberen Drittel, vorzugsweise im oberen Viertel, der Toilettenkabine 8 und insbesondere im oberen Drittel, vorzugsweise im oberen Viertel, des Eckpfostens 18, an dem die Beduftungseinrichtung 6 vorgesehen ist, angeordnet.

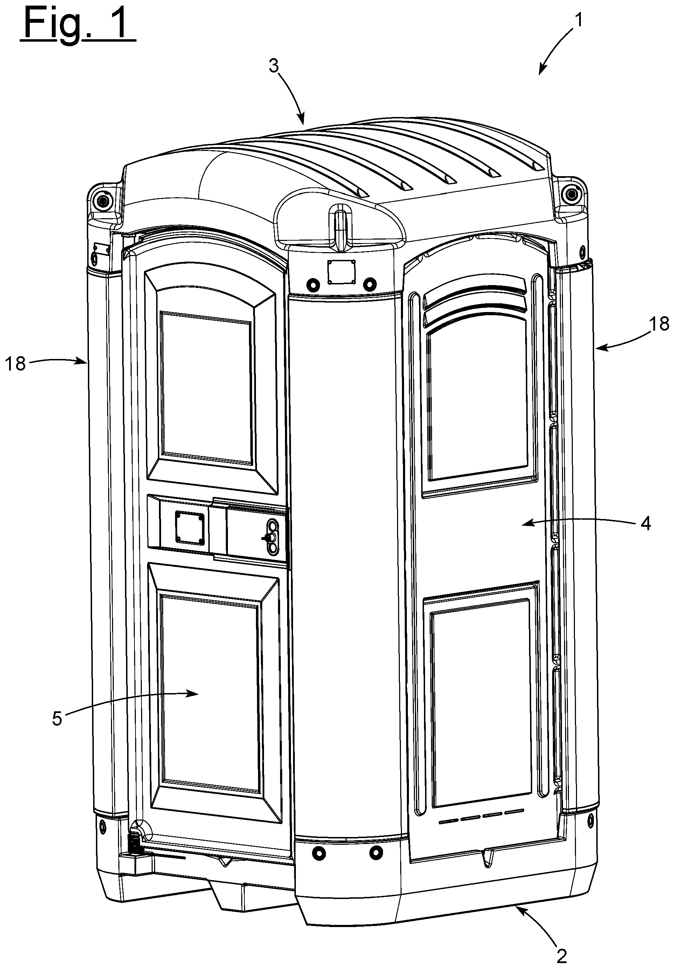

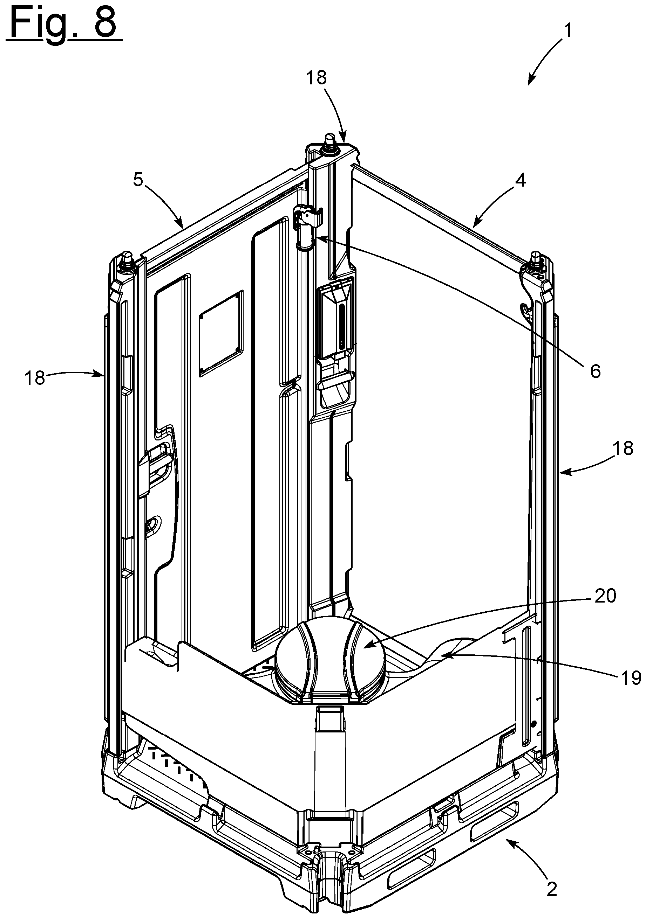

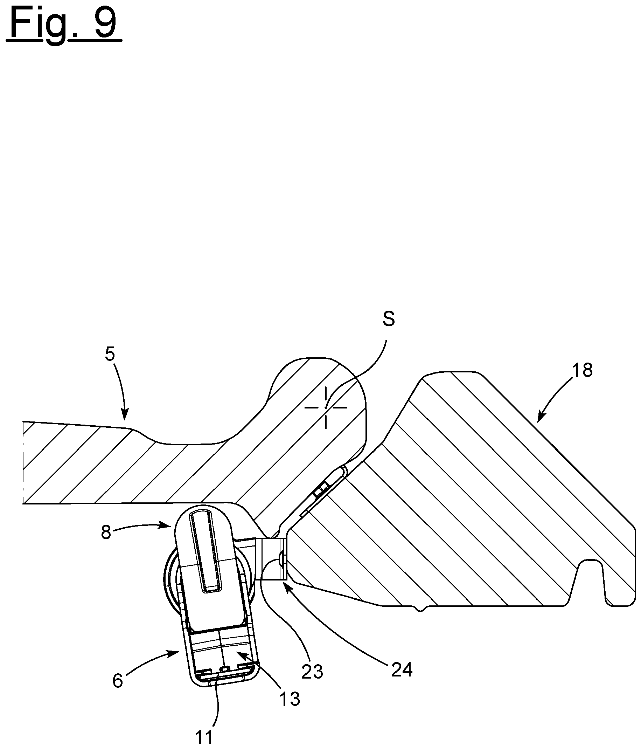

Sehr bevorzugt und im Ausführungsbeispiel erfolgt die Verbindung zwischen der Beduftungseinrichtung 6 und dem Eckpfosten 18 mittels Schraubelementen 23 (Figur 4). Im Ausführungsbeispiel und gemäß bevorzugter Ausführungsform sind zumindest drei, insbesondere drei, Schraubelemente 23 vorgesehen. Die Schraubelemente 23 durchsetzen bevorzugt und im Ausführungsbeispiel den unteren Teil 7b des Hauptkörpers 7 bzw. eine Wandung des unteren Teils 7b des Hauptkörpers 7 der Beduftungseinrichtung 6, sodass vorzugsweise und im Ausführungsbeispiel dieser untere Teil 7b des Hauptkörpers 7 der Beduftungseinrichtung 6 mit dem Eckpfosten 18 verschraubt wird. Gemäß sehr bevorzugter Ausführungsform und im Ausführungsbeispiel gemäß den Figuren 1 bis 3 wird der Hauptkörper 7 der Beduftungseinrichtung 6 direkt mit dem Eckpfosten 18 verschraubt. Gemäß einer weiteren bevorzugten Ausführungsform der Erfindung und gemäß den Figuren 8 und 9 ist ein Zwischenblech 24 zwischen dem Hauptkörper 7 der Beduftungseinrichtung 6 und dem Eckpfosten 18 angeordnet. Das Zwischenblech 24 dient vorzugsweise dazu, die Beduftungseinrichtung 6 im Hinblick auf die Zusammenwirkung mit der Kabinentür 5 auszurichten. Die Beduftungseinrichtung 6 ist somit an einem der Schwenkachse S der Kabinentür 5 zugeordneten Eckpfosten 18 der Sanitärkabine bzw. Toilettenkabine 1 angeordnet und insbesondere mit diesem Eckpfosten 18 verschraubt.

## Patentansprüche

1. Sanitärkabine, insbesondere Toilettenkabine (1), mit zumindest einem Bodenteil (2), zumindest einem Deckenteil (3), einer Mehrzahl von Seitenwandteilen (4) und mit zumindest einer verschwenkbaren Kabinentür (5), wobei die Sanitärkabine fernerhin zumindest eine Beduftungseinrichtung (6) mit zumindest einem Hauptkörper (7), zumindest einem - vorzugsweise als Auslösebügel (8) ausgestalteten - Auslöseelement und zumindest einem Duftmittelbehältnis (9) aufweist, wobei die Beduftungseinrichtung (6) mit der Maßgabe im Innenraum der Sanitärkabine angeordnet ist, dass das Auslöseelement beim Schließen und/oder Öffnen der Kabinentür (5) das Duftmittelbehältnis (9), vorzugsweise eine Abgabeeinrichtung des Duftmittelbehältnisses (9), derart beaufschlagt, dass von der Beduftungseinrichtung (6) ein Duftmittel in die Sanitärkabine abgebbar ist bzw. abgegeben wird und wobei dazu bevorzugt das Auslöseelement durch Zusammenwirkung mit der Kabinentür (5) betätigbar ist bzw. betätigt wird.

2. Sanitärkabine nach Anspruch 1, wobei die Abgabeeinrichtung als Sprüheinrichtung, insbesondere als Sprühkopf (10), ausgebildet ist und wobei das Duftmittel vorzugsweise in die Sanitärkabine sprühbar ist bzw. gesprüht wird.

3. Sanitärkabine nach einem der Ansprüche 1 oder 2, wobei das Auslöseelement, insbesondere der Auslösebügel (8), an dem Hauptkörper (7) der Beduftungseinrichtung (6) angeordnet ist, vorzugsweise angelenkt ist, und bevorzugt beim Schließen und/oder Öffnen der Kabinentür (5) - insbesondere durch Zusammenwirkung mit der Kabinentür (5) - von einer Warteposition in eine Auslöseposition überführbar ist bzw. überführt wird, vorzugsweise verschwenkbar ist bzw. verschwenkt wird, wobei das Auslöseelement, insbesondere der Auslösebügel (8), vorzugsweise bei der Überführung bzw. Verschwenkung von der Warteposition in die Auslöseposition das Duftmittelbehältnis, vorzugsweise die Abgabeeinrichtung des Duftmittelbehältnisses (9), beaufschlagt.

4. Sanitärkabine nach einem der Ansprüche 1 bis 3, wobei die Beduftungseinrichtung (6) zumindest ein Speicherelement, insbesondere einen Speicherfilz (11), aufweist, wobei Duftmittel, das aus der Abgabeeinrichtung abgegeben wird, in dem Speicherelement speicherbar ist bzw. gespeichert wird und wobei das Speicherelement vorzugsweise der Abgabeeinrichtung gegenüberliegend angeordnet ist, sodass die Abgabeeinrichtung bevorzugt zumindest einen Teil des Duftmittels gegen das Speicherelement abgibt, insbesondere sprüht.

5. Sanitärkabine nach einem der Ansprüche 1 bis 4, wobei der Hauptkörper (7) der Beduftungseinrichtung (6) zumindest einen Auffangabschnitt, insbesondere zumindest ein Auffangreservoir (13), aufweist, in dem von der Abgabeeinrichtung abgegebenes Duftmittel auffangbar ist bzw. aufgefangen wird und wobei der Auffangabschnitt, insbesondere das Auffangreservoir (13), vorzugsweise in der Duftmittelabgaberichtung zwischen der Abgabeeinrichtung und dem Speicherelement angeordnet ist.

6. Sanitärkabine nach einem der Ansprüche 1 bis 5, wobei das Auslöseelement, vorzugsweise der Auslösebügel (8), derart an dem Hauptkörper (7) der Beduftungseinrichtung (6) angeordnet ist, insbesondere angelenkt ist, dass es die Abgabeeinrichtung übergreift.

7. Sanitärkabine nach einem der Ansprüche 1 bis 6, wobei die Abgabeeinrichtung eine Feder, insbesondere eine Schraubenfeder, aufweist und wobei das Auslöseelement im Zuge der Beaufschlagung des Duftmittelbehältnisses (9), vorzugsweise der Abgabeeinrichtung, die Feder der Abgabeeinrichtung spannt und wobei das Auslöseelement beim Öffnen und/oder Schließen der Kabinentür (5) bevorzugt von der Rückstellkraft der Feder der Abgabeeinrichtung zurückstellbar ist bzw. zurückgestellt wird, sehr bevorzugt von der Auslöseposition in die Warteposition zurückgestellt wird.

8. Sanitärkabine nach einem der Ansprüche 1 bis 7, wobei die Beduftungseinrichtung (6) ein Rückstellelement, insbesondere eine Feder, aufweist, wobei das Rückstellelement an dem Hauptkörper (7), insbesondere an der Außenseite des Hauptkörpers (7), einerseits und an dem Auslöseelement andererseits angeordnet ist und wobei das Rückstellelement bevorzugt beim Schließen der Kabinentür (5) durch Zusammenwirkung des Auslöseelementes mit der Kabinentür (5) gespannt wird und beim Öffnen der Kabinentür entspannt und wobei sehr bevorzugt beim Öffnen der Kabinentür (5) das Auslöseelement durch die Rückstellkraft des Rückstellelementes betätigt wird und dabei das Duftmittelbehältnis (9), vorzugsweise die Abgabeeinrichtung des Duftmittelbehältnisses (9), derart beaufschlagt, dass von der Beduftungseinrichtung (6) ein Duftmittel in die Sanitärkabine abgebbar ist bzw. abgegeben wird.

9. Sanitärkabine nach einem der Ansprüche 1 bis 8, wobei der Hauptkörper (7) zumindest eine Aufnahmekammer (14) für das Duftmittelbehältnis (9) aufweist, wobei das Duftmittelbehältnis (9) vorzugsweise vollständig bzw. im Wesentlichen vollständig in der Aufnahmekammer (14) aufnehmbar ist und wobei der Hauptkörper (7) bevorzugt zumindest eine Aufnahmeöffnung (15) für die Einbringung des Duftmittelbehältnisses (9) in die Aufnahmekammer (14) aufweist, wobei die Aufnahmeöffnung (15) besonders bevorzugt im eingebrachten Zustand des Duftmittelbehältnisses (9) einem Boden des Duftmittelbehältnisses (9) zugeordnet ist.

10. Sanitärkabine nach Anspruch 9, wobei die Aufnahmeöffnung (15) mit zumindest einem Verschlussdeckel (16) verschließbar ist und wobei vorzugsweise ein Bajonettverschluss zwischen der Aufnahmeöffnung (15) und dem Verschlussdeckel (16) vorhanden ist.

11. Sanitärkabine nach einem der Ansprüche 1 bis 10, wobei die Beduftungseinrichtung (6) mit der Maßgabe in der Sanitärkabine angeordnet ist, insbesondere mit der Maßgabe an der die Kabinentür (5) aufweisenden Kabinenseite angeordnet ist, dass das Auslöseelement, vorzugsweise der Auslösebügel (8), bei geöffneter Kabinentür (5) in eine Türöffnung (17) der Sanitärkabine hineinragt und beim Schließen der Kabinentür (5), bevorzugt von der Kabinentür (5), aus der Türöffnung (17) herausgedrückt wird und dabei insbesondere von der Warteposition in die Auslöseposition oder von der Auslöseposition in die Warteposition überführt bzw. verschwenkt wird.

12. Sanitärkabine nach einem der Ansprüche 1 bis 11, wobei die Sanitärkabine zumindest drei, vorzugsweise zumindest vier, Eckpfosten (18) aufweist, die das Bodenteil (2) mit dem Deckenteil (3) verbinden und wobei die zumindest eine Beduftungseinrichtung (6) vorzugsweise an einem der Eckpfosten (18) angeordnet ist, bevorzugt an einem der Schwenkachse S der Kabinentür (5) zugeordneten Eckpfosten (18) angeordnet ist.

13. Sanitärkabine nach einem der Ansprüche 1 bis 12, wobei die zumindest eine Beduftungseinrichtung (6) an einem Türrahmen der Kabinentür (5) angeordnet ist.

14. Sanitärkabine nach einem der Ansprüche 1 bis 13, wobei in der Sanitärkabine zumindest ein Fäkalientank (19) und/oder zumindest eine Sanitäreinrichtung, insbesondere zumindest ein WC-Becken (20) und/oder zumindest ein Urinal und/oder zumindest ein Handwaschbecken, angeordnet ist/sind.

15. Sanitärkabine nach einem der Ansprüche 1 bis 14, wobei die Sanitärkabine zumindest teilweise, vorzugsweise vollständig bzw. im Wesentlichen vollständig, aus zumindest einem Kunststoff, bevorzugt aus zumindest einem thermoplastischen Kunststoff, sehr bevorzugt aus Polypropylen und/oder Polyethylen, ausgebildet ist und wobei bevorzugt zumindest das Bodenteil (2) und/oder das Deckenteil (3) und/oder die Seitenwandteile (4) und/oder die Eckpfosten (18) und/oder die Kabinentür (5) und/oder die zumindest eine Sanitäreinrichtung und/oder die zumindest eine Beduftungseinrichtung (6), aus dem zumindest einen thermoplastischen Kunststoff ausgebildet ist/sind, sehr bevorzugt aus Polyethylen ausgebildet ist/sind und besonders bevorzugt aus Polyethylen besteht/bestehen bzw. im Wesentlichen besteht/bestehen.

16. Verfahren zum Betrieb einer Sanitärkabine, insbesondere einer Toilettenkabine (1), nach einem der Ansprüche 1 bis 15, wobei beim Schließen und/oder Öffnen der verschwenkbaren Kabinentür (5) das Auslöseelement, insbesondere der Auslösebügel (8), der Beduftungseinrichtung (6) das Duftmittelbehältnis (9), vorzugsweise eine Abgabeeinrichtung des Duftmittelbehältnisses (9), derart beaufschlagt, dass von der Beduftungseinrichtung (6) ein Duftmittel in die Sanitärkabine abgeben wird und wobei dazu bevorzugt das Auslöseelement durch Zusammenwirkung mit der Kabinentür (5) betätigt wird.

## Geänderte Patentansprüche

### Geänderte Patentansprüche gemäss Regel 137(2) EPÜ.

1. Sanitärkabine, insbesondere Toilettenkabine (1), mit zumindest einem Bodenteil (2), zumindest einem Deckenteil (3), einer Mehrzahl von Seitenwandteilen (4) und mit zumindest einer verschwenkbaren Kabinentür (5), wobei die Sanitärkabine fernerhin zumindest eine Beduftungseinrichtung (6) mit zumindest einem Hauptkörper (7), zumindest einem - vorzugsweise als Auslösebügel (8) ausgestalteten - Auslöseelement und zumindest einem Duftmittelbehältnis (9) aufweist, wobei die Beduftungseinrichtung (6) mit der Maßgabe im Innenraum der Sanitärkabine angeordnet ist, dass das Auslöseelement beim Schließen und/oder Öffnen der Kabinentür (5) das Duftmittelbehältnis (9), vorzugsweise eine Abgabeeinrichtung des Duftmittelbehältnisses (9), derart beaufschlagt, dass von der Beduftungseinrichtung (6) ein Duftmittel in die Sanitärkabine abgebbar ist bzw. abgegeben wird, wobei dazu bevorzugt das Auslöseelement durch Zusammenwirkung mit der Kabinentür (5) betätigbar ist bzw. betätigt wird
und wobei die Beduftungseinrichtung (6) mit der Maßgabe in der Sanitärkabine angeordnet ist, dass das Auslöseelement, vorzugsweise der Auslösebügel (8), bei geöffneter Kabinentür (5) in eine Türöffnung (17) der Sanitärkabine hineinragt und beim Schließen der Kabinentür (5), bevorzugt von der Kabinentür (5), aus der Türöffnung (17) herausgedrückt wird.

2. Sanitärkabine nach Anspruch 1, wobei die Abgabeeinrichtung als Sprüheinrichtung, insbesondere als Sprühkopf (10), ausgebildet ist und wobei das Duftmittel vorzugsweise in die Sanitärkabine sprühbar ist bzw. gesprüht wird.

3. Sanitärkabine nach einem der Ansprüche 1 oder 2, wobei das Auslöseelement, insbesondere der Auslösebügel (8), an dem Hauptkörper (7) der Beduftungseinrichtung (6) angeordnet ist, vorzugsweise angelenkt ist, und bevorzugt beim Schließen und/oder Öffnen der Kabinentür (5) - insbesondere durch Zusammenwirkung mit der Kabinentür (5) - von einer Warteposition in eine Auslöseposition überführbar ist bzw. überführt wird, vorzugsweise verschwenkbar ist bzw. verschwenkt wird, wobei das Auslöseelement, insbesondere der Auslösebügel (8), vorzugsweise bei der Überführung bzw. Verschwenkung von der Warteposition in die Auslöseposition das Duftmittelbehältnis, vorzugsweise die Abgabeeinrichtung des Duftmittelbehältnisses (9), beaufschlagt.

4. Sanitärkabine nach einem der Ansprüche 1 bis 3, wobei die Beduftungseinrichtung (6) zumindest ein Speicherelement, insbesondere einen Speicherfilz (11), aufweist, wobei Duftmittel, das aus der Abgabeeinrichtung abgegeben wird, in dem Speicherelement speicherbar ist bzw. gespeichert wird und wobei das Speicherelement vorzugsweise der Abgabeeinrichtung gegenüberliegend angeordnet ist, sodass die Abgabeeinrichtung bevorzugt zumindest einen Teil des Duftmittels gegen das Speicherelement abgibt, insbesondere sprüht.

5. Sanitärkabine nach einem der Ansprüche 1 bis 4, wobei der Hauptkörper (7) der Beduftungseinrichtung (6) zumindest einen Auffangabschnitt, insbesondere zumindest ein Auffangreservoir (13), aufweist, in dem von der Abgabeeinrichtung abgegebenes Duftmittel auffangbar ist bzw. aufgefangen wird und wobei der Auffangabschnitt, insbesondere das Auffangreservoir (13), vorzugsweise in der Duftmittelabgaberichtung zwischen der Abgabeeinrichtung und dem Speicherelement angeordnet ist.

6. Sanitärkabine nach einem der Ansprüche 1 bis 5, wobei das Auslöseelement, vorzugsweise der Auslösebügel (8), derart an dem Hauptkörper (7) der Beduftungseinrichtung (6) angeordnet ist, insbesondere angelenkt ist, dass es die Abgabeeinrichtung übergreift.

7. Sanitärkabine nach einem der Ansprüche 1 bis 6, wobei die Abgabeeinrichtung eine Feder, insbesondere eine Schraubenfeder, aufweist und wobei das Auslöseelement im Zuge der Beaufschlagung des Duftmittelbehältnisses (9), vorzugsweise der Abgabeeinrichtung, die Feder der Abgabeeinrichtung spannt und wobei das Auslöseelement beim Öffnen und/oder Schließen der Kabinentür (5) bevorzugt von der Rückstellkraft der Feder der Abgabeeinrichtung zurückstellbar ist bzw. zurückgestellt wird, sehr bevorzugt von der Auslöseposition in die Warteposition zurückgestellt wird.

8. Sanitärkabine nach einem der Ansprüche 1 bis 7, wobei die Beduftungseinrichtung (6) ein Rückstellelement, insbesondere eine Feder, aufweist, wobei das Rückstellelement an dem Hauptkörper (7), insbesondere an der Außenseite des Hauptkörpers (7), einerseits und an dem Auslöseelement andererseits angeordnet ist und wobei das Rückstellelement bevorzugt beim Schließen der Kabinentür (5) durch Zusammenwirkung des Auslöseelementes mit der Kabinentür (5) gespannt wird und beim Öffnen der Kabinentür entspannt und wobei sehr bevorzugt beim Öffnen der Kabinentür (5) das Auslöseelement durch die Rückstellkraft des Rückstellelementes betätigt wird und dabei das Duftmittelbehältnis (9), vorzugsweise die Abgabeeinrichtung des Duftmittelbehältnisses (9), derart beaufschlagt, dass von der Beduftungseinrichtung (6) ein Duftmittel in die Sanitärkabine abgebbar ist bzw. abgegeben wird.

9. Sanitärkabine nach einem der Ansprüche 1 bis 8, wobei der Hauptkörper (7) zumindest eine Aufnahmekammer (14) für das Duftmittelbehältnis (9) aufweist, wobei das Duftmittelbehältnis (9) vorzugsweise vollständig bzw. im Wesentlichen vollständig in der Aufnahmekammer (14) aufnehmbar ist und wobei der Hauptkörper (7) bevorzugt zumindest eine Aufnahmeöffnung (15) für die Einbringung des Duftmittelbehältnisses (9) in die Aufnahmekammer (14) aufweist, wobei die Aufnahmeöffnung (15) besonders bevorzugt im eingebrachten Zustand des Duftmittelbehältnisses (9) einem Boden des Duftmittelbehältnisses (9) zugeordnet ist.

10. Sanitärkabine nach Anspruch 9, wobei die Aufnahmeöffnung (15) mit zumindest einem Verschlussdeckel (16) verschließbar ist und wobei vorzugsweise ein Bajonettverschluss zwischen der Aufnahmeöffnung (15) und dem Verschlussdeckel (16) vorhanden ist.

11. Sanitärkabine nach einem der Ansprüche 1 bis 10, wobei die Sanitärkabine zumindest drei, vorzugsweise zumindest vier, Eckpfosten (18) aufweist, die das Bodenteil (2) mit dem Deckenteil (3) verbinden und wobei die zumindest eine Beduftungseinrichtung (6) vorzugsweise an einem der Eckpfosten (18) angeordnet ist, bevorzugt an einem der Schwenkachse S der Kabinentür (5) zugeordneten Eckpfosten (18) angeordnet ist.

12. Sanitärkabine nach einem der Ansprüche 1 bis 11, wobei die zumindest eine Beduftungseinrichtung (6) an einem Türrahmen der Kabinentür (5) angeordnet ist.

13. Sanitärkabine nach einem der Ansprüche 1 bis 12, wobei in der Sanitärkabine zumindest ein Fäkalientank (19) und/oder zumindest eine Sanitäreinrichtung, insbesondere zumindest ein WC-Becken (20) und/oder zumindest ein Urinal und/oder zumindest ein Handwaschbecken, angeordnet ist/sind.

14. Sanitärkabine nach einem der Ansprüche 1 bis 13, wobei die Sanitärkabine zumindest teilweise, vorzugsweise vollständig bzw. im Wesentlichen vollständig, aus zumindest einem Kunststoff, bevorzugt aus zumindest einem thermoplastischen Kunststoff, sehr bevorzugt aus Polypropylen und/oder Polyethylen, ausgebildet ist und wobei bevorzugt zumindest das Bodenteil (2) und/oder das Deckenteil (3) und/oder die Seitenwandteile (4) und/oder die Eckpfosten (18) und/oder die Kabinentür (5) und/oder die zumindest eine Sanitäreinrichtung und/oder die zumindest eine Beduftungseinrichtung (6), aus dem zumindest einen thermoplastischen Kunststoff ausgebildet ist/sind, sehr bevorzugt aus Polyethylen ausgebildet ist/sind und besonders bevorzugt aus Polyethylen besteht/bestehen bzw. im Wesentlichen besteht/bestehen.

15. Verfahren zum Betrieb einer Sanitärkabine, insbesondere einer Toilettenkabine (1), nach einem der Ansprüche 1 bis 14, wobei beim Schließen und/oder Öffnen der verschwenkbaren Kabinentür (5) das Auslöseelement, insbesondere der Auslösebügel (8), der Beduftungseinrichtung (6) das Duftmittelbehältnis (9), vorzugsweise eine Abgabeeinrichtung des Duftmittelbehältnisses (9), derart beaufschlagt, dass von der Beduftungseinrichtung (6) ein Duftmittel in die Sanitärkabine abgeben wird und wobei dazu bevorzugt das Auslöseelement durch Zusammenwirkung mit der Kabinentür (5) betätigt wird.
